# EUROPEAN PATENT SPECIFICATION

(11) **EP 4 206 236 B1**
(45) Date of publication and mention of the grant of the patent: **24.06.2026**
(21) Application number: 22214908.0
(22) Date of filing: 20.12.2022
(51) Int. Cl.: C08F 8/14, A61K 8/81, A61Q 5/02

(54) **COMPOUNDS, THEIR SYNTHESIS AND COMPOSITIONS COMPRISING THE COMPOUNDS FOR ENHANCED DEPOSITION OF POLYMERS ONTO HAIR SURFACES**
VERBINDUNGEN, DEREN SYNTHESE UND ZUSAMMENSETZUNGEN MIT DEN VERBINDUNGEN ZUR VERBESSERTEN ABLAGERUNG VON POLYMEREN AUF HAAROBERFLÄCHEN
COMPOSÉS, LEURS SYNTHÈSES ET COMPOSITIONS COMPRENANT LES COMPOSÉS POUR UN DÉPÔT AMÉLIORÉ DE POLYMÈRES SUR DES SURFACES CAPILLAIRES

(30) Priority: 28.12.2021 EP 21217959
(43) Date of publication of application: 05.07.2023
(73) Proprietor: Kao Corporation, Chuo-ku Tokyo 103-8210 (JP)
(72) Inventor: Breakspear, Steven, 64297 Darmstadt (DE); Nöcker, Bernd, 64297 Darmstadt (DE); Weitzel, Johanna, 64297 Darmstadt (DE); Paez, Julieta, 66123 Saarbrücken (DE); Pearson, Samuel, 66123 Saarbrücken (DE); Brück, Stefan, 66123 Saarbrücken (DE); Campo, Aranzazu del, 55128 Mainz (DE)
(74) Representative: Hoffmann Eitle

(56) References cited:
- WO-A1-2008/025671
- CN-A- 111 073 160

## Description

The present invention is for polymers derivatized with an organic deposition-enhancing group for their enhanced deposition onto keratin fibers, especially human hair. The deposition-enhancing group is a di-hydroxyl phenolic compound with a spacer alkyl group bound to a reactive amine or carboxyl group, which reacts with one of the hydroxyl, carboxyl, primary and secondary amine groups on the polymeric backbone. The invention is on the method of synthesizing such compounds and compositions comprising the compounds.

Hair compositions have been used for many decades, primarily for improving hair properties. They are usually based on cationic compounds, which are adsorbed onto the hair surface via electrostatic interactions. Although the achieved results are generally satisfactory, the obtained effect lasts only for a very short time, as the adsorbed cationic compounds are washed off, mostly with a single shampooing, due to complexing with the anionic surfactants in the shampoo compositions. This requires the consumer to use conditioners almost after every wash cycle, which is often found time consuming, uneconomical and not environmental friendly.

There have been attempts to solve the problem by providing conditioners, which provide long lasting effect. One of the methods has been to use oily substances at high concentrations. Another method has been treating hair with cationic polymers and with heat, which produces long lasting conditioning effects, which may last for 5 to 10 washes.

Another proposed method in EP2056788 Unilever is using dihydroxy benzoic acid esterified poly(hydroxyethyl acrylate or poly(hydroxyethyl methacrylate) polymers for the long lasting deposition of the polymers. The document only discloses dihydroxy phenolic compounds, which carries the reactive carboxyl group without a spacer alkyl chain.

In further publications such as WO2015/193302, WO2005/056708, WO2006/026556, US5110915, GB1583173, US2012016390, US4212645 and US010515077 catechol derivatized polymers of various kinds have been suggested for various purposes among which cosmetic hair conditioners are also suggested.

The above publications realize certain level of long lasting deposition onto the substrates; however, the deposition yield and their long lastingness are not satisfactory.

The inventors of the present invention unexpectedly found out that a polymeric backbone attached to a deposition enhancing group of dihydroxy phenol carrying a reactive carboxyl or primary amine group attached with a spacer alkyl / alkylene chain, deposited onto the hair surface at a much higher rate and it also exhibits very much improved long-lasting deposition.

Thus, the first object of the present invention is a compound comprising a polymeric backbone comprising one or more of reactive hydroxyl, carboxyl and/or primary or secondary amine groups and a deposition-enhancing group, according to the general structure 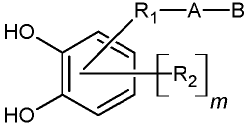 wherein
R₁ is C₁ to C₄ alkyl or C₂ to C₄ alkyl, which may be saturated or unsaturated and/or substituted with one or more groups excluding a primary, secondary or tertiary amine group, and a COOH group or C₃ to C₄ branched alkyl, which may be saturated or unsaturated and/or substituted with one or more groups excluding a primary, secondary or tertiary amine group, and a COOH group,
A is a carboxyl or a primary amine group,
B is a polymeric backbone carrying one or more of hydroxyl, carboxyl and/or primary or secondary amine group, R₂ is OH, C₁ to C₄ alkyl, C₂ to C₄ alkyl, which may be saturated or unsaturated and/or substituted with one or more groups excluding a primary, secondary or tertiary amine group, and a COOH group and C₃ to C₄ branched alkyl, which may be saturated or unsaturated and/or substituted with one or more hydroxyl groups excluding a primary, secondary or tertiary amine group, and a COOH group and m is 0 to 3,
wherein 1 to 50% of the total reactive hydroxyl, carboxyl and/or primary or secondary amine groups on the polymeric backbone are occupied with deposition enhancing group,
with the condition that the A is a carboxyl group when the polymeric backbone B has primary and/or secondary amine groups as the reactive group or hydroxyl group and A is primary amine when the polymeric backbone B has carboxyl groups as the reactive group.

The second object is a method of synthesizing the compounds of the above general structure, comprising the steps of:
a- the polymer and the deposition enhancing dihydroxy compound are dissolved in a solvent or mixture of solvents, preferably selected from dichloromethane, N,N-dimethyl formamide, water and their mixture, and
b- reacted for a period of 1 h to 7 days, preferably 6 hrs to 5 days at a temperature in the range of 20 to 60°C, in the presence of one or more coupling agents selected from EDC, NHS, PyBOP, HBTU, HOBt, DCC, DIC and their mixture, and optionally a catalyst selected from DIPEA and DMAP, and
c- optionally, the derivatized polymer is precipitated by changing the solvent to a solvent wherein the derivatized polymer is insoluble, preferably selected from Diethylether and/or acetone, and
d- the derivatized polymer is dissolved in a solvent or mixture of solvents preferably selected from dichloromethane, N,N-dimethyl formamide, water and their mixture and dialyzed against methanol or 1mM hydrochloric acid solution, and
e- the dry purified derivatized polymer is obtained by freeze drying,
with the conditions,
when the to be derivatized polymer comprises carboxyl groups as the reactive group, the coupling agent is selected from EDC and/or NHS, and the reaction was carried out without a catalyst,
when the to be derivatized polymer comprises primary or secondary amine groups as the reactive group, the coupling agent is selected from PyBOP, HBTU, HOBt, DCC, DIC, EDC and their mixture and the catalyst is DIPEA, and
when to be derivatized polymer comprises hydroxyl groups as the reactive group, the coupling agent is DCC, and the catalyst is DMAP.

The third object is for a cosmetic composition for keratin fibers, especially for human hair, comprising one or more of the polymeric compounds with attached deposition-enhancing group according to the above general structure.

The fourth object is the method of treating keratin fibers, especially human hair, wherein the composition comprising one or more of the polymeric compounds with attached deposition-enhancing group according to the above general structure is applied onto hair, and rinsed off from hair, after leaving on the hair for an appropriate period of time to allow deposition of the polymers onto hair.

The fifth object of the invention is a kit comprising one or more cosmetic products for keratin fibers, especially human hair, comprising one or more of the polymeric compounds with attached deposition enhancing group according to the above general structure.

The deposition-enhancing group is attached to a polymeric backbone comprising one or more of reactive hydroxyl, carboxyl, or primary or secondary amine groups. The nature of the polymeric backbone is not important but the polymer must bear at least one of the said reactive groups and be soluble in a solvent or solvent mixture, preferably selected from water, ethanol, dichloromethane, N,N-dimethyl formamide and their mixtures, in order to carry out the synthesis. It should be noted that the polymer must not be soluble in all the solvents listed above, it is satisfactory if the polymer is soluble in one of them or only in special mixtures of these solvents.

The polymeric backbone is preferably selected from the polymers comprising the monomer units of acrylic acid, methacrylic acid, ethacrylic acid, methyl acrylate, ethyl acrylate, n-butyl acrylate, iso-butyl acrylate, t-butyl acrylate, 2-ethylhexyl acrylate, decyl acrylate, octyl acrylate, methyl methacrylate, ethyl methacrylate, n-butyl methacrylate, iso-butyl methacrylate, t-butyl methacrylate, 2-ethylhexyl methacrylate, decyl methacrylate, methyl ethacrylate, ethyl ethacrylate, n-butyl ethacrylate, iso-butyl ethacrylate, t-butyl ethacrylate, 2-ethylhexyl ethacrylate, decyl ethacrylate, 2,3-dihydroxypropyl acrylate, 2,3-dihydroxypropyl methacrylate, 2-hydroxyethyl acrylate, 2-hydroxypropyl acrylate, hydroxypropyl methacrylate, glyceryl monoacrylate, glyceryl monoethacrylate, glycidyl methacrylate, glycidyl acrylate, acrylamide, methacrylamide, ethacrylamide, N-methyl acrylamide, N,N-dimethyl acrylamide, N,N-dimethyl methacrylamide, N-ethyl acrylamide, N-isopropyl acrylamide, N-butyl acrylamide, N-t-butyl acrylamide, N,N-di-n-butyl acrylamide, N,N-diethylacrylamide, N-octyl acrylamide, N-octadecyl acrylamide, N,N-diethylacrylamide, N-phenyl acrylamide, N-methyl methacrylamide, N-ethyl methacrylamide, N-dodecyl methacrylamide, N,N-dimethylaminoethyl acrylamide, quaternized N,N-dimethylaminoethyl acrylamide, N,N-dimethylaminoethyl methacrylamide, quaternized N,N-dimethylaminoethyl methacrylamide, N,N-dimethylaminoethyl acrylate, N,N-dimethylaminoethyl methacrylate, quaternized N,N-dimethyl-aminoethyl acrylate, quaternized N,N-dimethylaminoethyl methacrylate, 2-hydroxyethyl acrylate, 2-hydroxyethyl methacrylate, 2-hydroxyethyl ethacrylate, glyceryl acrylate, 2-methoxyethyl acrylate, 2-methoxyethyl methacrylate, 2-methoxyethyl ethacrylate, 2-ethoxyethyl acrylate, 2-ethoxyethyl methacrylate, 2-ethoxyethyl ethacrylate, maleic acid, maleic anhydride and its half esters, fumaric acid, itaconic acid, itaconic anhydride and its half esters, crotonic acid, angelic acid, diallyldimethyl ammonium chloride, vinyl pyrrolidone, vinyl imidazole, methyl vinyl ether, methyl vinyl ketone, maleimide, vinyl pyridine, vinyl furan, styrene sulphonate, allyl alcohol, allyl citrate, allyl tartrate, vinyl acetate, vinyl alcohol, and mixtures thereof, silicones with isopropyl, propyl or butyl amino propyl amine groups which may be grafted with oxazoline comprising groups, and heteropolysaccharide gum, such as xanthan gum.

The suitable non-limiting examples of the polymeric backbones commercially available are Polyquaternium-7, Polyquaternium-22, Polyquaternium-39, polysilicone-9, and amodimethicones available from various suppliers which may vary in detailed chemical structures.

The polymeric backbone is preferably selected from the polymers not comprising caprolactam group.

In the preferred form of the present invention, the deposition-enhancing group of the compound according to the above general structure comprises as R₁ a C₂ alkyl (ethyl) group, and does not comprise any R₂ so that m is 0.

Suitable compounds as the deposition-enhancing group are dihydroxyphenylethylamine, dihydroxyphenyl propionic acid, norepinephrine, hydroxytyrosol (compounds from natural olive oil extracts etc), and caffeic acid. The most preferred are dihydroxyphenylethylamine and dihydroxyphenyl propionic acid.

It is clear that not all of the reactive groups present on the polymeric backbone must be occupied with the deposition-enhancing group in order to promote the deposition and the long lastingness of the polymer on the hair. The deposition enhancing and long lastingness are observed with the polymeric compounds having as low as 1% of the reactive groups occupied with the deposition-enhancing group. This is, however, not a fully satisfactory deposition enhancement and its long lastingness is limited. Therefore, in a preferred embodiment, the degree of derivatization is in the range of 2 to 50%, preferably 5 to 40%, more preferably 7.5 to 30% and most preferably, it is 10 to 25% of the available reactive groups on the polymeric backbone.

The polymers particularly preferred are Polyhydroxyethylacrylate, Polyquaternium-22 which is a copolymer of Diallyldimethyl Ammonium Chloride and Acrylic Acid, the polysilicone polymers commercially available from Kao Corporation and known with CTFA adopted name Polysilicone-9.

The polymeric backbones are attached to the deposition-enhancing group with a derivatization reaction. The derivatization reaction takes place in a solution of the polymer and the deposition-enhancing group in the above referred solvents in the presence of one or more coupling agents listed above and optionally one or more catalysts, which may be selected from DIPEA and DMAP for a period 1h to 7 days preferably 6 hrs to 5 days at the temperature range 20 to 60°C. The reaction product is precipitated by changing the solvent to a solvent wherein the derivatized polymer is insoluble. The precipitated polymer is dissolved again in a solvent used during the reaction and the solution is dialyzed against methanol or 1 mM hydrochloric acid solution for removing excess amount of agents other than derivatized polymer. Finally, the derivatized polymer is obtained by freeze drying. The derivatization was confirmed by ¹H NMR analysis with disappearance or decrease of the peaks from the starting material and the appearance / increase of the peaks for the reaction products. The examples explain further in detail.

Particularly, the derivatization of the polymers is carried out as outlined below, in three ways, and the characterization and quantification as defined below.

### Grafting of dihydroxyphenolic compound to polymers bearing carboxyl pendant groups by amidation reactions

Dihydroxyphenol derivatisation (C = left part of structure comprising the dihydroxy phenyl ring and the linker group R) of polymers (B) containing carboxyl groups (Merquat 280, A1000, Fixomer A-30, Xanthan) were done by amidation reaction forming an amide bond (A).

Dopamine hydrochloride (DA), which bears an amine functional group, was used as the dihydroxyphenol reactant (C). DA feeds between 10 and 100 wt% of starting polymer A were used in order to have variants with different dihydroxy phenyl contents.

Amidation reactions using standard coupling agents converted the COOH groups into activated esters, which were attacked by the nucleophilic amine group of DA to form amide bonds. Amidation reactions were performed in dichloromethane (DCM), N,N-dimethylformamide (DMF), water, or mixtures thereof at temperatures ranging from room temperature to 40 °C and for reaction times ranging from 12 h to 5 days. The preferred derivatisation of Merquat 280 was performed in DMF/water (2:1 v/v) for 16 h at room temperature under nitrogen atmosphere. In reactions where dry organic solvents were used, a dry nitrogen atmosphere was also employed. The coupling agent 1-ethyl-3-(3-dimethylaminopropyl) carbodiimide (EDC) was used for activation of the COOH pendant groups on the polymer. For functionalisation of the polymer Xanthan, a mixture of EDC and N-hydroxysuccinimide (NHS) was used for activation of the COOH groups.

Purification was performed by precipitation first into a miscible solvent (typically diethyl ether or acetone) which did not dissolve the derivatized polymer, but dissolved unreacted educts and activating agent by-products. Acetone was preferred as miscible solvent for precipitation of DA-modified Merquat 280.

Precipitated polymers were then dissolved in water and dialysed against acidified water to remove trace impurities. Acidified water (typically 1 mmol/l hydrochloric acid) was used for DA-modified Merquat 280 to prevent oxidation of catechol groups during dialysis. Dialysis was performed in a membrane tube several days until no trace impurity of dopamine were detected as absorbance at λ 280 nm in UV/Vis spectrum of dialysis water.

The purified polymer derivatives were isolated from the dialysed aqueous polymer solutions by freeze-drying to give the final polymers.

The catechol-modified polymer derivatives were analysed by ¹H NMR spectroscopy in a Bruker 300 MHz spectrometer.

### Grafting of Dihydroxyphenolic compound to polymers bearing amine pendant groups by amidation reactions

Dihydroxyphenol derivatives of polymers containing amine pendant groups (KAO OX40, KAO OS71, Dow Corning 2-2078, Dow Corning 2-8566) were synthesized by amidation reaction with one of the two COOH-bearing Dihydroxyphenol compounds dihydrocaffeic acid (DHCA, alternate short name is DHPPA for 3-(3,4-dihydroxyphenyl)propionic acid) or 3,4-dihydroxybenzoic acid (DHBA).

Different coupling agents were used, including bromotripyrrolidinophosphonium hexafluorophosphate (PyBOP), O-(benzotriazol-1-yl)-*N,N,N',N*'-tetramethyluronium hexafluorophosphate (HBTU)/1-hydroxybenzotriazol (HOBt) and the carbodiimides *N,N'*-dicyclohexylcarbodiimide (DCC), *N,N*'-diisopropylcarbodiimide (DIC) and EDC.

In some cases *N,N*-diisopropylethylamine (DIPEA) was used as catalyst.

The amidation reaction conditions were similar to the carboxyl-amine cases.

Dialysis was performed typically in methanol.

The purified polymer derivatives were isolated from the dialysed aqueous polymer solutions by freeze drying to give the final polymers.

The polymer derivatives were analysed by quantitative ¹H NMR spectroscopy in a Bruker 300 MHz spectrometer.

### Grafting of Dihydroxyphenol compound to polymers bearing hydroxyl groups by esterification reactions

Dihydroxyphenol derivatisation of pendant hydroxyl groups from poly(2-hydroxyethyl acrylate) (PHEA) was done by Steglich esterification reaction. Bonding group B is an ester group in this case.

COOH-bearing catechols DHCA and DHBA were grafted onto the polymer.

Dicyclohexylcarbodiimide (DCC) was used as a coupling reagent. In the preferred esterification conditions, 4-dimethylaminopyridine (DMAP) was also added to the reaction mixture as catalyst.

Esterifications were typically performed in organic solvent (DCM, DMF) at room temperature to 40 °C for 1 to 5 days.

Crude purification was performed by precipitation into a miscible solvent (cold diethyl ether) which does not dissolve the derivatized polymer but dissolve unreacted educts and activating agent by-products. The crude product was twice purified by reprecipitation from methanol into diethyl ether.

For final purification, the precipitate was dissolved in water and dialysed against acidified water to remove trace impurities. Acidified water (1 mmol/l hydrochloric acid) was used to prevent oxidation of catechol groups. Dialysis was performed in a membrane tube with regular replacement of the acidifid water for several days until no trace impurity of catechol (DHCA, DHBA) was detected as absorbance at λ 280 nm in the UV/Vis spectrum of the dialysis medium.

The purified polymer derivatives were isolated from the dialysed aqueous polymer solutions by freeze drying to give the final polymers.

The polymer derivatives were analysed by quantitative ¹H NMR spectroscopy in a Bruker 300 MHz spectrometer.

One or more catalysator may be employed for promoting the reaction speed, preferably selected from diisopropyl carbodiimide (DIC), Dicyclohexylcarbodiimid (DCC), Bromotripyrrolidinophosphonium hexafluorophosphate (PyProP^{®}), (2-(1*H-*benzotriazol-1-yl)-1,1,3,3-tetramethyluronium hexafluorophosphate (HBTU), Benzotriazol-1-ol (HOBT).

The characterisation of the derivation is carried out by 1H NMR spectroscopy as described below.

### NMR-METHOD 1: Weight percent of dihydroxyphenol compoud determined by ¹H NMR spectroscopy

NMR-METHOD 1 was used for polymers where molecular structures were not fully disclosed or spectra had too broad signals for integration of repeating units: Merquat 280, OX40-DHCA, A1000-DA, Xanthan-DA, Fixomer A-30. The method gives the degree of derivatisation in wt%.

In all NMR samples prepared for NMR-METHOD 1, 1.0 mg/ml of sodium 2,2-dimethyl-2-silapentane-5-sulfonate-d6 (DSS) was added for calibration of the chemical shift axis to 0 ppm, and to act as internal standard for quantifying the degree of derivatisation. Integration limits for the DSS peak were set from 0.25 to - 0.25 ppm.

Standard DA solutions were first prepared by dissolving 0.5 to 3.5 mg (in 0.5 mg increments) of DA in 1.000 ml D₂O containing 1.0 mg/ml DSS. A ¹H NMR spectrum for each solution was obtained in a 300 MHz Bruker FT-NMR instrument using number of scans (NS) = 128 and delay between scans (D1) = 30 s.

The integral of the aromatic proton signals of the catechol units with chemical shift 7.5 to 6.4 ppm, denoted I_{aromatic}, was divided by the integral of the internal standard I_{standard} to give a normalized integral I_{aromatic,norm}. A plot of DA mass in the samples versus the corresponding I_{aromatic,norm} values formed a calibration curve for subsequent analysis of the polymers.

Samples of catechol-derivatized polymers were then prepared at 12.0 to 20.0 mg polymer/ml D₂O also containing 1.0 mg/ml DSS.

The normalized integral I_{aromatic,norm} was measured in the same way as with DA. The equivalent DA mass corresponding to this normalized integral I_{aromatic,norm} was taken from the DA calibration curve.

The degree of derivatisation in wt% was then calculated as the mass of DA divided by the mass of catechol-derivatized polymer in the NMR sample.

### NMR-METHOD 2: Degree of derivatisation in Mole percent of dihydroxyphenol per polymer repeat unit determined by ¹H NMR spectroscopy

NMR-METHOD 2 was used for polymers where the molecular structure was known, and signals were narrow enough to be used as internal standard for accurate integration, for example in PHEA. This method gives the degree of derivatisation in mol%.

Samples of the purified polymers were dissolved in deuterated solvent (D₂O or methanol-d4) to give a concentration of ca. 50 mg/ml. Spectra were recorded identically to NMR-METHOD 1. The chemical shift axis was calibrated using the deuterated solvent signal.

Firstly, the aromatic proton signals I_{aromatic} in the chemical shift region of 7.2 to 6.4 ppm were integrated. This region corresponds to 3 protons from the grafted catechol group (DHCA, DHBA). Secondly, the integral of protons contained in each repeat unit of the base polymer (I_{base}) was determined from the same spectrum. The chemical shift of the repeating unit was known from spectrum of the ungrafted polymer. For PHEA, the proton signals from the 2 x methylene (CH₂) groups (4H) at 4.5 to 3.5 ppm were used for I_{base}.

The degree of derivatisation was then calculated as (I_{aromatic} / 3) / (I_{base} / X) x 100 mol%, where X = number of protons giving the signal in the repeat unit of the base polymer (X = 4 for PHEA ).

Particularly preferred polymers comprising the deposition enhancing groups are the following compounds: 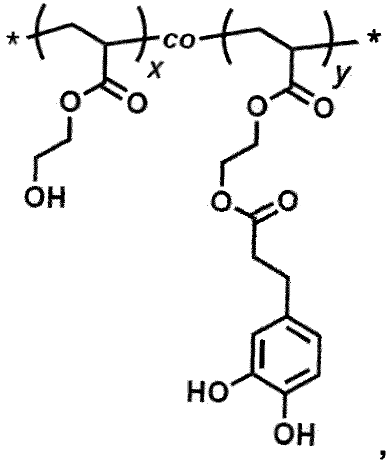 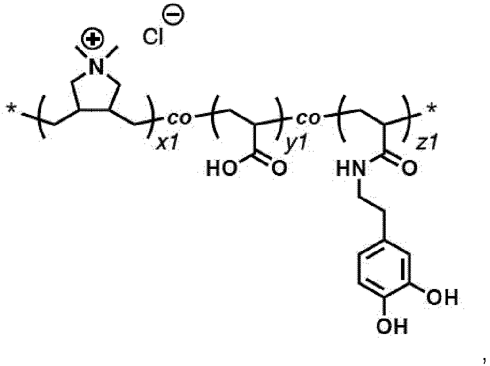 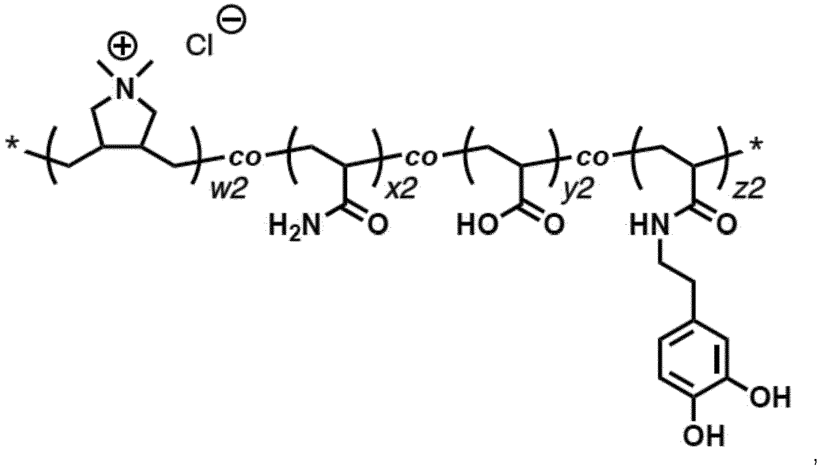 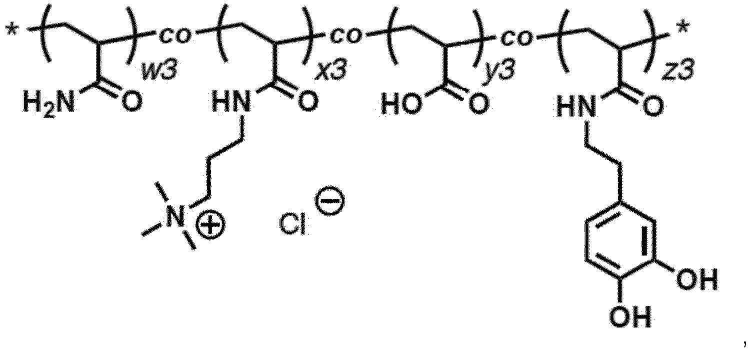 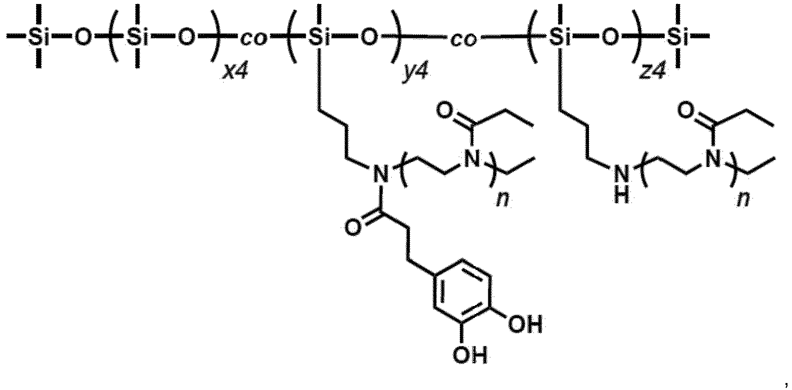 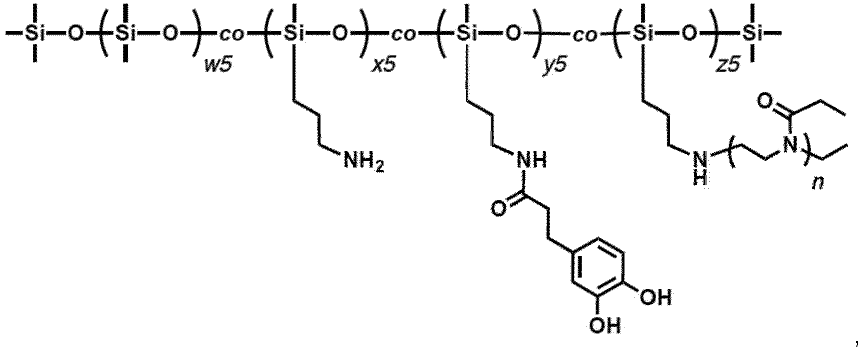 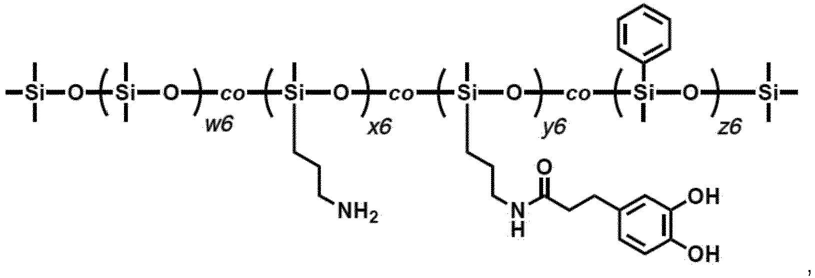 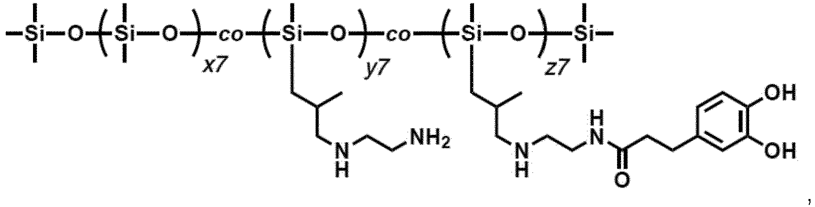 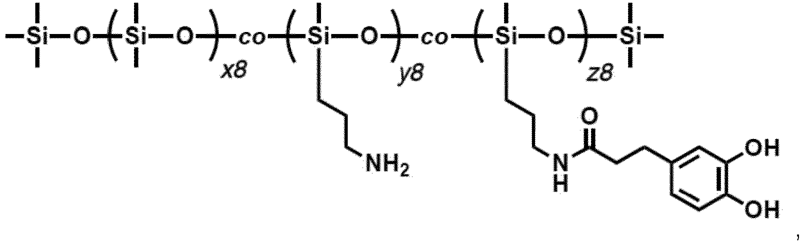 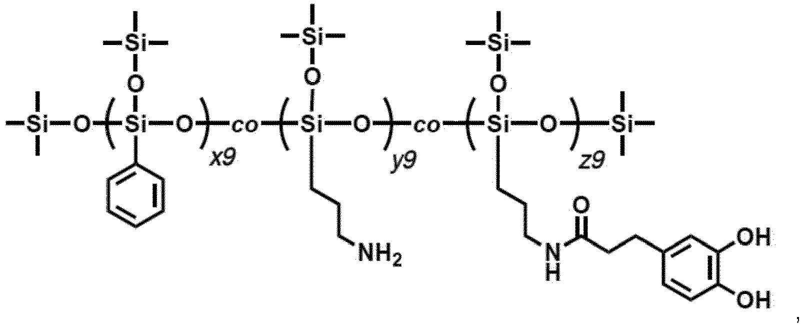 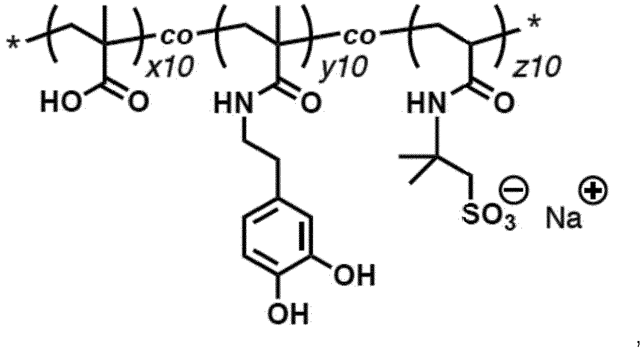 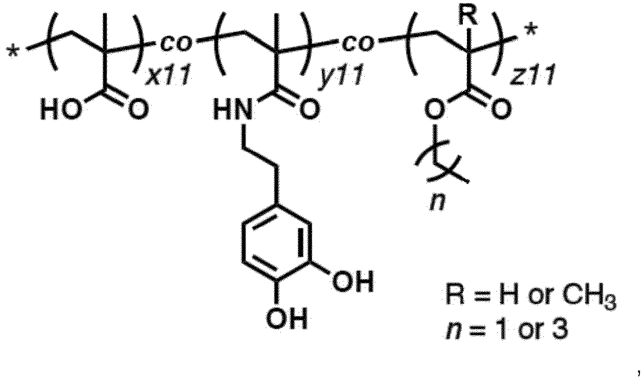 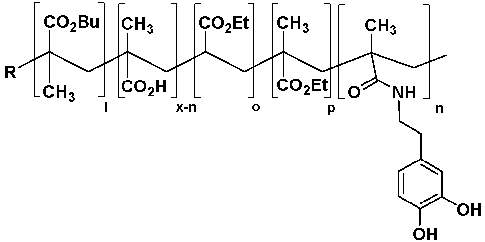 and

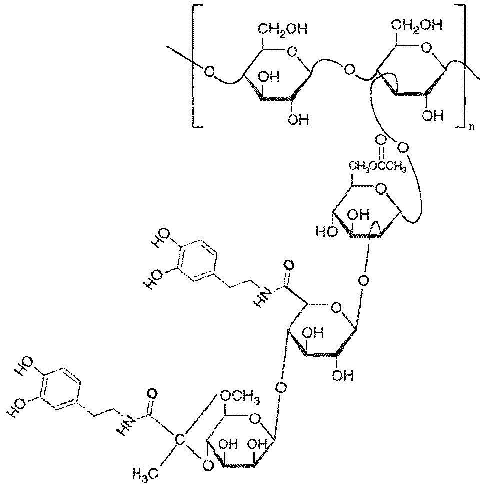

The cosmetic compositions for keratin fibers, especially human hair, comprising the polymers with the deposition-enhancing group may be in the form of shampoos, conditioners, hair sprays, gels, colouring compositions, permanent and/or semipermanent shaping compositions, styling compositions, aerosols, and lotions. The compositions may be aqueous, aqueous - alcoholic, or alcoholic.

The compositions comprise the novel derivatized polymers at a concentration in the range of 0.1 to 5%, preferably 0.1 to 4%, more preferably 0.15 to 3% and most preferably 0.2 to 2.5% by weight, calculated to the total of the composition.

In a preferred embodiment of the present invention, the compositions are aqueous compositions, which may comprise additional compounds customarily found in hair cosmetic compositions.

The compositions may be used as a leave in or a rinse off compositions depending on the type of the composition mentioned above. The preferred method of use is the rinse off, wherein the composition is applied onto wet or dry hair and rinsed off after certain period of time which may be as short as a few minutes and as long as days.

The hair conditioning compositions are preferably aqueous compositions and comprising at least 50% by weight, water calculated to the total of the composition.

On the other hand, the composition for hair styling may be ethanolic or water ethanolic compositions comprising water and ethanol at various levels.

The aqueous conditioners may comprise additional compounds such as fatty alcohols, surfactants, which may be selected from anionic, cationic, nonionic and amphoteric ones, additional conditioners such as cationic polymers, natural and synthetic oils, cationic surfactants which may as well be quaternary ammonium surfactants, polyols, natural ingredients which may be added as extracts, UV filter to protect hair fiber and hair colour, further organic solvents other than the mentioned ones, fragrances, preservatives.

Further in detail, the compositions may comprise one or more surfactants either as emulsifiers, especially in the compositions of emulsion form and used mainly after shampoo conditioning compositions, or in cleansing compositions as foaming and cleansing compounds.

Aqueous cleansing composition comprises at least one surfactant selected from anionic, non-ionic and amphoteric surfactants at a concentration range of 5 to 50%, preferably 5 to 40% and more preferably 7.5 to 30%, and most preferably 10 to 25% by weight, calculated to the total composition.

In an embodiment, aqueous cleansing composition comprises at least one anionic, at least one nonionic surfactant. More preferably, the composition further comprises additionally at least one amphoteric surfactant.

In principle any anionic surfactant is suitable for cleansing compositions. Nonlimiting examples are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof with a C₈-C₂₀-alkyl group, preferably a C₁₂-C₁₄-alkyl group and with number of EO units in the range of 1 to 20 and sodium, potassium, magnesium and ammonium salts thereof. Suitable alkyl amido polyether carboxylates are as well with a C₈-C₂₀-alkyl group and with number of EO units in the range of 1 to 10 and sodium, potassium, magnesium and ammonium salts thereof.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, cocoyl glutamate preferably in form of the water-soluble alkali or ammonium, in particular the sodium salts thereof, preferably in admixture with the above-named anionic surfactants.

Commonly used and preferred anionic surfactants are those of alkyl ether sulphates such as lauryl ether sulphate and aminocarboxylic acids such as lauroyl glutamate sodium salt.

Further surfactants in the shampoo compositions are nonionic surfactants especially in admixture with anionic surfactants. Especially suited are alkyl polyglucosides of the general formula

R₄-O-(R₅O)ₙ-Zₓ,

wherein R₄ is an alkyl group with 8 to 18 carbon atoms, R₅ is an ethylene or propylene group, Z is a saccharide group with 5 to 6 carbon atoms, n is a number from 0 to 10 and x is a number between 1 and 5.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components may be present, for example, long-chain fatty acid dialkanolamides, such as coco fatty acid diethanolamide and myristic fatty acid diethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16". The average degree of ethoxylation thereby ranges between about 2.5 and about 25, preferably about 10 and about 20.

As further surfactant component, the composition comprises amphoteric surfactants, for example in an amount from about 0.5 % to about 15 %, preferably from about 1 % to about 10 %, by weight, calculated to the total composition. It has especially been suited that addition of amphoteric surfactants enhances foam feeling in terms of creaminess, foam volume and as well as skin compatibility is improved. For achieving milder formulations, anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable. The preferred amphoteric surfactants are alkyl betaines such as lauryl betaine and alkyl amido betaines such as cocamidopropyl betaine.

In preferred embodiment, aqueous cleansing composition comprises at least one anionic surfactant especially of alkyl ether sulphate type, at least one amphoteric surfactant especially alkyl amido alkyl betaine type and at least one non-ionic surfactant especially an alkyl polyglucoside. In a further preferred form of the present invention, in addition to the above mentioned surfactant the composition comprises additionally acyl amido carboxylic acid surfactant especially sodium lauroyl glutamate.

The compositions may comprise one or more fatty alcohol having alkyl chain length of 8 to 24 C atoms, which may be saturated or unsaturated and straight of branched at a concentration in the range of 0.1 to 25% by weight calculated to the total of the composition. The concentration of the fatty alcohol is relatively low in cleansing compositions, but the compositions used as conditioner after cleansing hair comprises relatively high concentrations of fatty alcohol.

Suitable non-limiting preferred examples are cetyl alcohol, stearyl alcohol, myristyl alcohol, cetearyl alcohol, oleyl alcohol and behenyl alcohol.

Compositions may comprise further one or more cationic polymers as conditioning compounds in cleansing compositions as well as after cleansing conditioners. Suitable are well-known polyquaternium compounds which may be exemplified as Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

Further suitable cationic polymers are especially those of cationic cellulose types known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum and its derivatives known with INCI name Caesalpinia spinosa hydroxypropyltrimonium chloride. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The preferred cationic polymers especially in cleansing compositions are those of cationic cellulose derivatives, cationic guar gum derivatives, cationic Caesalpinia spinosa gum derivatives, polyquaternium 6, polyquaternium 7, polyquaternium 67 and polyquaternium 70.

Cationic surfactants are suitable conditioners and also suitable emulsifiers especially in emulsion conditioners. Suitable cationic surfactants are according to the general structure 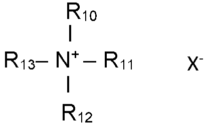 where R₁₀ is a saturated or unsaturated, branched or non-branched alkyl chain with 8-22 C atoms or

R₁₄CO NH (CH₂)ₙ

where R₁₄ is saturated or unsaturated, branched or non-branched alkyl chain with 7-21 C atoms and n has typical value of 1 - 4 or

R₁₅CO O (CH₂)ₙ

where R₁₅ is saturated or unsaturated, branched or non-branched alkyl chain with 7-21 C atoms and n has typical value of 1 - 4, and and R₁₁ is a saturated or unsaturated, branched, or non-branched alkyl chain with 1-22 C atoms or

R₁₄CO NH(CH₂)ₙ

where R₁₄ is saturated or unsaturated, branched or non-branched alkyl chain with 7-21 C atoms and n has typical value of 1 - 4 or

R₁₅CO O (CH₂)ₙ

where R₁₅ is saturated or unsaturated, branched or non-branched alkyl chain with 7-21 C atoms and n has typical value of 1 - 4, and
R₁₂ and R₁₃ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Non-limiting examples to suitable cationic surfactants are cetyl trimethyl ammonium chloride, myristoyl trimethyl ammonium chloride, behentrimonium chloride, stearyl trimethyl ammonium chloride, dimethyl distearyl ammonium chloride and stearamidopropyldimethyl ammonium chloride.

The composition may comprise one or more synthetic or natural oil (s). In principal, any oil allowed for cosmetic use is suitable for the compositions of the present invention.

Oils are those of synthetic and of natural ones. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum are suitably contained within the scope of the present invention, It should as well be noted that compositions may contain mixture of one or more natural oils and mineral oil.

Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401, DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions.

Concentration of one or more oil in the compositions may vary in the range of 0.01 and 10%, preferably 0.05 and 7.5% more preferably 0.1 and 5% and most preferably 0.25 and 2.5% by weight calculated to total composition.

The composition may comprise polyols at a concentration of 0.1 to 15%, preferably 0.2 to 10%, more preferably 0.5 to 7.5% by weight calculated to the total concentration . The most preferred ones are glycerine, propylene glycols, butylene glycol, panthenol and hexylene glycol.

The compositions according to the invention may also comprise further agents, such as proteins, for example bamboo protein, and protein hydrolyzates and polypeptides, e.g. keratin hydrolyzates, collagen hydrolyzates of the type "Nutrilan^{®}" or elastin hydrolyzates, as well as, in particular vegetable, optionally cationized protein hydrolyzates, for example "Gluadin^{®}".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition . Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon^{®}" products, "Herbasol^{R} ", "Sedaplant^{R}" and "Hexaplant^{R}". Extracts and the preparation thereof are also described in "Hagers Handbuch der pharmazeutischen Praxis". 4^{th} Ed..

Compositions may comprise UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysilicone-15.

The suitable amount of the UV-absorber ranges from about 0.01 % to 1% by weight, calculated to the total composition . Attention should be paid to the stability and solubility especially when using UV filter as salts, e.g. anionic UV filter salts.

Further, the compositions may comprise one or more direct dye for colouring hair. Suitable direct dyes are cationic, anionic, neutral dyes and their mixtures as available commercially from various suppliers and used mainly in semi-permanent hair coloration.

Non-limiting examples to cationic dyes are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

Concentration of direct dyes in the compositions of the present invention is within the range of 0.001 to 5%, preferably 0.01 to 3% and more preferably 0.05 to 2%, and most preferably 0.1 to 1% by weight calculated to total composition, calculated to total composition.

Compositions for styling hair applied after cleansing and conditioning hair may comprise one or more film forming polymers selected from the anionic, non-ionic, cationic and/or amphoteric or zwitterionic ones, especially the non-ionic, anionic and amphoteric polymers.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG and Advantage LC-E from ISP.

Natural non-ionic polymers are as well suitable for the composition of the present invention. Those are such as cellulose, chitosan, guar gum, neutralised shellac and their derivatives.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer^{®}"; copolymers from methacryloyl ethyl betaine and alkyl methacrylates of the type "Yukaformer^{®}", e.g.. the butyl methacrylate copolymer "Yukaformer^{®} Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from USA 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez^{®} AN or ES". These polymers may also be partly esterified, as for example, "Gantrez^{®} ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn^{®}"; sodium acrylate/vinyl alcohol copolymers of the type "Hydagen^{®} F", sodium polystyrene sulfonate, e.g.. "Flexan^{®} 130"; ethyl acrylate/acrylic acid/N-tert.-butyl acrylamide copolymers of the type "Ultrahold^{®}"; vinyl pyrrolidone/vinyl acetate/itaconic acid copolymers, acrylic acid/acrylamide copolymers or the sodium salts thereof.

Further suitable anionic polymers are Acrylate copolymers available under trade name Salcare SC 81, PEG/PPG 25/25 dimethicone / acrylate copolymer available under trade name Luviflex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Cationic polymers disclosed above as conditioning polymers may as well be used as film forming polymers.

The concentration of the film forming polymers is in the range of 1 to 20% by weight calculated to the total composition.

In case of an aerosol type of styling product is desired, then composition comprises additionally one or more propellant at a concentration of 5 to 60%, preferably 5 to 50% more preferably 10 to 50% by weight calculated to total composition. Suitable propellants are lower alkanes such as n-butane, i-butane, propane, butane or their mixtures, as well as dimethylether (DME) either alone or in mixture with lower alkanes. Further suitable propellants are fluorinated hydrocarbons such as 1,1-difluoro ethane or tetrafluoroethane or their mixtures with each other, carbon dioxide and nitogen or their mixtures with the above mentioned propellants. Preferred are lower alkanes such as propane, butane, n-butane, i-butane, and their mixtures and their mixture with DME at a alkane to DME weight ratio 10:1 to 1:10.

The compositions may comprise further compounds customarily found in a cosmetic composition such as preservative, fragrances, acids and basis for adjusting the pH.

The following are the examples to demonstrate the invention but not to limit it.

### Example 1

### Synthesis of Dihydroxy phenyl proponic acid (DHPPA) grafted polyhydroxyethylacrylate (PHEA)

**WITH DMAP CATALYST:** PHEA (3.2 g), DHPPA (1.50 g), and DMAP (53 mg) were dissolved in 15 ml of dry DMF in a 100 ml flask together with PTFE coated magnetic stirrer bar. The flask was closed with a rubber septum. This solution was cooled to 0 °C with stirring and a solution of DCC (1.7 g) in DMF (5 ml) was added with syringe through septum dropwise over a period of 10 min. The reaction mixture was allowed to warm to room temperature and was stirred for a further 5 days. After this time, the solution was filtered with a glass frit and vacuum to remove dicyclohexylurea by-product and the resulting clear solution was poured dropwise in 200 ml cold diethyl ether to precipitate the crude product. The crude product was twice purified by reprecipitation from methanol into diethyl ether.

For further purification a 2 wt% aqueous solution of reprecipitation product was filled in regenerated cellulose membrane tube (molecular weight cut-off, MWCO = 3500 Da) and the closed tube was dialysed against deionized water for 24 h with 3 x water changes. The pure polymer was isolated by freeze-drying.

Samples of the purified polymers were dissolved in deuterated solvent (D₂O or methanol-d4) to give a concentration of ca. 50 mg/ml. A ¹H NMR spectrum for each solution was obtained in a 300 MHz Bruker FT-NMR instrument using number of scans (NS) = 128 and delay between scans (D1) = 30 s.. The chemical shift axis was calibrated using the deuterated solvent signal.

Firstly, the aromatic proton signals I_{aromatic} in the chemical shift region of 7.2 to 6.4 ppm were integrated. This region corresponds to 3 protons from the grafted catechol group (DHPPA, DHBA). Secondly, the integral of protons contained in each repeat unit of the base polymer (I_{base}) was determined from the same spectrum. The chemical shift of the repeating unit was known from spectrum of the ungrafted polymer. For PHEA, the proton signals from the 2 x methylene (CH₂) groups (4H) at 4.5 to 3.5 ppm were used for I_{base}.

The degree of derivatisation was then calculated as (I_{aromatic} / 3) / (I_{base} / X) x 100 mol%, where X = number of protons giving the signal in the repeat unit of the base polymer (X = 4 for PHEA ).

The DHPPA grafted PHEA was determined to have a degree of derivatisation of 17.2 mol%.

**WITHOUT DMAP CATALYST:** PHEA (3.2 g) and DHPPA (1.50 g) were dissolved in 15 ml of dry DMF in a 100 ml flask together with PTFE coated magnetic stirrer bar. The flask was closed with a rubber septum. This solution was cooled to 0 °C with stirring and a solution of DCC (1.7 g) in DMF (5 ml) was added with syringe through septum dropwise over a period of 10 min. The reaction mixture was allowed to warm to room temperature and was stirred for a further 48 hours. After this time, the solution was filtered with a glass frit and vacuum to remove dicyclohexylurea by-product and the resulting clear solution was poured dropwise in 200 ml cold diethyl ether to precipitate the crude product. The crude product was twice purified by reprecipitation from methanol into diethyl ether.

Samples of the purified polymers were dissolved in deuterated solvent (D₂O or methanol-d4) to give a concentration of ca. 50 mg/ml. A ¹H NMR spectrum for each solution was obtained in a 300 MHz Bruker FT-NMR instrument using number of scans (NS) = 128 and delay between scans (D1) = 30 s.. The chemical shift axis was calibrated using the deuterated solvent signal.

Firstly, the aromatic proton signals I_{aromatic} in the chemical shift region of 7.2 to 6.4 ppm were integrated. This region corresponds to 3 protons from the grafted catechol group (DHPPA, DHBA). Secondly, the integral of protons contained in each repeat unit of the base polymer (I_{base}) was determined from the same spectrum. The chemical shift of the repeating unit was known from spectrum of the ungrafted polymer. For PHEA, the proton signals from the 2 x methylene (CH₂) groups (4H) at 4.5 to 3.5 ppm were used for I_{base}**.**

The degree of derivatisation was then calculated as (I_{aromatic} / 3) / (I_{base} / X) x 100 mol%, where X = number of protons giving the signal in the repeat unit of the base polymer (X = 4 for PHEA ).

The DHPPA content in the precipitated polymer was 13.1 mol%.

### Example 2 (comparative example not within the scope of the claims)

### Synthesis of Dihydroxy benzoic acid (DHBA) grafted polyhydroxyethylacrylate (PHEA)

**WITH DMAP CATALYST:** PHEA (3.2 g), DHBA (1.27 g), and DMAP (53 mg) were dissolved in 15 ml of dry DMF in a 100 ml flask together with PTFE coated magnetic stirrer bar. The flask was closed with a rubber septum. This solution was cooled to 0 °C with stirring and a solution of DCC (1.7 g) in DMF (5 ml) was added with syringe through septum dropwise over a period of 10 min. The reaction mixture was allowed to warm to room temperature and was stirred for a further 5 days. After this time, the solution was filtered with a glass frit and vacuum to remove dicyclohexylurea by-product and the resulting clear solution was poured dropwise in 200 ml cold diethyl ether to precipitate the crude product. The crude product was twice purified by reprecipitation from methanol into diethyl ether.

For further purification a 2 wt% aqueous solution of reprecipitation product was filled in regenerated cellulose membrane tube (molecular weight cut-off, MWCO = 3500 Da) and the closed tube was dialysed against deionized water for 24 h with 3 x water changes. The pure polymer was isolated by freeze-drying.

Samples of the purified polymers were dissolved in deuterated solvent (D₂O or methanol-d4) to give a concentration of ca. 50 mg/ml. A ¹H NMR spectrum for each solution was obtained in a 300 MHz Bruker FT-NMR instrument using number of scans (NS) = 128 and delay between scans (D1) = 30 s.. The chemical shift axis was calibrated using the deuterated solvent signal.

Firstly, the aromatic proton signals I_{aromatic} in the chemical shift region of 7.2 to 6.4 ppm were integrated. This region corresponds to 3 protons from the grafted catechol group (DHCA, DHBA). Secondly, the integral of protons contained in each repeat unit of the base polymer (I_{base}) was determined from the same spectrum. The chemical shift of the repeating unit was known from spectrum of the ungrafted polymer. For PHEA, the proton signals from the 2 x methylene (CH₂) groups (4H) at 4.5 to 3.5 ppm were used for I_{base}**.**

The degree of derivatisation was then calculated as (I_{aromatic} / 3) / (I_{base} / X) x 100 mol%, where X = number of protons giving the signal in the repeat unit of the base polymer (X = 4 for PHEA ).

The DHBA grafted PHEA was determined to have a degree of derivatisation of 0.3 mol%.

**WITHOUT DMAP:** PHEA (3.2 g) and DHBA (1.27g), were dissolved in 15 ml of dry DMF in a 100 ml flask together with PTFE coated magnetic stirrer bar. The flask was closed with a rubber septum. This solution was cooled to 0 °C with stirring and a solution of DCC (1.7 g) in DMF (5 ml) was added with syringe through septum dropwise over a period of 10 min. The reaction mixture was allowed to warm to room temperature and was stirred for a further 48 hours. After this time, the solution was filtered with a glass frit and vacuum to remove dicyclohexylurea by-product and the resulting clear solution was poured dropwise in 200 ml cold diethyl ether to precipitate the crude product. The crude product was twice purified by reprecipitation from methanol into diethyl ether.

Samples of the purified polymers were dissolved in deuterated solvent (D₂O or methanol-d4) to give a concentration of ca. 50 mg/ml. A ¹H NMR spectrum for each solution was obtained in a 300 MHz Bruker FT-NMR instrument using number of scans (NS) = 128 and delay between scans (D1) = 30 s. The chemical shift axis was calibrated using the deuterated solvent signal.

Firstly, the aromatic proton signals I_{aromatic} in the chemical shift region of 7.2 to 6.4 ppm were integrated. This region corresponds to 3 protons from the grafted catechol group (DHCA, DHBA). Secondly, the integral of protons contained in each repeat unit of the base polymer (I_{base}) was determined from the same spectrum. The chemical shift of the repeating unit was known from spectrum of the ungrafted polymer. For PHEA, the proton signals from the 2 x methylene (CH₂) groups (4H) at 4.5 to 3.5 ppm were used for I_{base}.

The degree of derivatisation was then calculated as (I_{aromatic} / 3) / (I_{base} / X) x 100 mol%, where X = number of protons giving the signal in the repeat unit of the base polymer (X = 4 for PHEA ).

The DHBA content in the precipitated polymer was 16.9 mol%.

### Example 3

### Deposition of the polymers obtained under examples 1 and 2 onto hair.

The deposition of the polymers onto hair was analysed gravimetrically, as follows:
Hair-filled columns were produced from glass pipettes (230 mm length, upper body OD: 7,1 mm, wall thickness: 0,53 mm, OD tip: 1,50 mm) which were packed with 350 mg (±1%) cut hair fibers with tweezers. The hair fibers were cut from 10 hair tresses (source KAO) as ca. 10 mm long hair pieces and the pieces were mixed thoroughly and taken randomly.

Stock solutions of each of the polymers (20 mg/ml in ethanol) were prepared as well as an aqueous buffer solution (0.1 mol/l NH₃/NH₄Cl pH 9.5).

In the test a hair-filled column was placed upright with tip down in a holder and a clean, long-neck pre-weighed glass vial 1 was placed under the column.

A deposition solution was freshly prepared by mixing 1.000 ml of polymer stock solution (or 1.000 ml of ethanol in the case of the background elution test resp.) with 1.000 ml buffer solution. 1.000 ml of the deposition solution was transferred into the hair-containing column with a pipette.

The column was closed with Parafilm and the deposition solution was left stationary in contact with the hair for 10 minutes.

Then Parafilm seals were removed and the solution that flowed from the column was collected for 10 minutes in pre-weighed glass vial 1. Glass vial 1 was closed with a tight cap.

For the rinsing step, a clean, long-neck pre-weighed glass vial 2 was placed under the column. 1.000 ml ethanol was added in the top of the column and the rinsing liquid passing through the column was collected in glass vial 2. Rinsing was repeated two times more, using 3.000 ml of ethanol in total. Glass vial 2 was closed with a tight cap..

For determining the dry mass of rinsed material recovered in glass vials 1 and 2, the vials were opened and placed on a 40 °C heating plate and a gentle, dry N₂ stream was blown with a needle carefully over the liquid surface for 1 h to evaporate all liquids. The heat bath was then removed and the vials were cooled down under dry N₂ stream before weighing on a 0.1 mg resolution lab balance to determine the dry mass of rinsed material.

In order to eliminate contribution of the any dissolved material from blank hair fibers, the above was also carried out without polymer and M_{eluted, blank} and m_{rinsed, blank} were determined.

The polymer uptaken (mᵤₚₜₐₖₑ) was calculated as the difference between m_{eluted} and m_{eluted, blank}. The uptake percentage was calculated by dividing the mᵤₚₜₐₖₑ by mᵢₙᵢₜᵢₐₗ and multiplying it by 100.

The substantiated polymer (m_{substantive}) amount was calculated by extracting the difference between (m_{eluted} - m_{eluted, blank}) and (m_{rinsed} - m_{rinsed, blank}) from the mᵢₙᵢₜᵢₐₗ. The percentage of substantivitity is the ratio between m_{substantive} and mᵢₙᵢₜᵢₐₗ.

As a control, the same as above was carried out with a polymer solution without the deposition enhancing groups.

The following results were obtained.

| | Uptake/% | Substantivity/% |
|---|---|---|
| Polymer itself | 55 | 6 |
| Polymer + DHPPA | 94 | 80 |
| Polymer + DHBA | 52 | 16 |

From the above results, it is clear that the uptake of the polymer itself and polymer plus DHBA are similar, but the uptake of the polymer with DHPPA is significantly higher. The results also show that the substantivity of the polymer is greatly enhanced for the polymer with DHPPA.

### Example 4

### Determination of effect of polymer deposition and durability of effect

Sample tresses, treated with the different polymers, were subjected to a three-point bending test and the maximum force required to deform the tresses by 20mm was measured.

Hair tresses to be tested were stored in a climate-controlled atmosphere of 20°C and 65% relative humidity for 16 hours prior to testing. Measurements were performed three times per tress and the average force calculated.

The hair tresses were then washed by hand and the maximum force of deformation was remeasured, as above, after three, five, seven, and ten washing steps. Each washing step consisted of prewetting the hair with warm water, followed by the application of ca. 2g of a shampoo (sold under the brand name Kerasilk Purifying Shampoo, Kao Germany GmbH) and working the shampoo into the tress for ca. 30 seconds, following by rubbing the hair tress under a stream of warm water for a further 90 seconds. The hair tresses were then blow-dried for one hour at a medium heat.

| | Maximum Force (mN) | | | | |
|---|---|---|---|---|---|
| | Initial | 3x washed | 5x washed | 7x washed | 10x washed |
| Untreated hair | 65 | 64 | 65 | 65 | 64 |
| Polymer + DHPPA | 295 | 221 | 188 | 118 | 65 |
| Polymer + DHBA | 65 | 65 | 64 | 65 | 65 |

The results above show that the initial force to deform the tress treated with polymer plus DHPPA is significantly higher than that treated with polymer plus DHBA, where the initial force of deformation was the same as untreated hair. It can also be seen that the effect of polymer plus DHPPA lasted for up to ten washes, before the deformation force returned to that of untreated hair. The force to deform the hair tress treated with polymer plus DHBA remained essentially constant throughout the experiment, indicating no effect to be present and, therefore, no durability of effect could be established.

### Example 5

### Synthesis of DA grafted Merquat 280

Merquat 280 (M280, freeze-dried from commercial ca. 40 wt% solution, 2.0 g) was filled in a 100 ml round-bottom flask. Water (10 ml) was added and the polymer was dissolved with magnetic stirring using a PTFE coated magnetic stirrer bar. DMF (99+%, extra pure, 20 ml) was added under stirring. A septum was put on the flask and N₂ was passed through the solution with long needle for 30 minutes. EDC (1.2 g, 60 wt% feed) was dissolved in a small vial with N₂-purged DMF/water (2:1 v/v, 3 ml) and the solution was transferred with a syringe into the M280 flask. Within 5 min after EDC addition, dopamine hydrochloride (1.2 g, 60 wt% feed) was dissolved in N₂-purged DMF/water (2:1 v/v, 3 ml) and transferred with a syringe through the septum into the M280/EDC flask. A N₂ balloon was fitted on the flask and the reaction mixture was stirred for 16 h.

For precipitation of the crude polymer, a 50 ml Falcon tube was filled with acetone (30 ml) and 10 ml of the reaction mixture was added slowly with a dripping funnel. The flacon tube was tightly closed and shaken until the polymer precipitated as a continuous mass. The supernatant was decanted off, and the solid was washed three times with acetone (30 ml each wash). The washed solid was collected in a 250 ml flask and residual acetone was evaporated using an air stream overnight.

After solvent removal, the solid material was ground into smaller particles using an agate mortar and pestle. A mixture of acetone/water (90:10 v/v, 50 ml) was added to the solid particles, and the particle suspension was stirred for 12 h using a PTFE coated magnet stirrer bar in order to extract trace impurities from the product. The suspension was recovered by filtration using MN619de filter paper (type very slow). Solid crude product was dried under vacuum.

A final purification by dialysis was done in acidified water. Solid crude product (2 g) was dissolved in 100 ml of 1 mmol/l hydrochloric acid and filled in regenerated cellulose dialysis tube (MWCO 3500 Da). The closed tube was put in a dialysis vessel filled with 7 L of 0.001 mol/l hydrochloric acid and dialysed with change of the dialysis medium each 8 h until there was no absorbance signal at λ = 280 nm in the UV/Vis spectrum of the dialysis medium (typically 2-3 days). A final dialysis step was added against DI water for 8 h. The contents of the dialysis tube were transferred in a 500 ml flask and freeze-dried to give the final product.

### Example 6

### Deposition of DA grafted Merquat 280 onto hair

The deposition of grafted M280 and non-grafted M280 onto each washed hair tress was performed by thoroughly wetting the tress for 20 s with tap-water at 38°C, and squeezing out the excess water between thumb and finger wearing nitrile gloves. The hair tress was immersed for 10 min in 15 ml alkaline buffer (0.1 mol/l NH₃/NH₄Cl, pH 9.5) at room temperature. Excess buffer was squeezed out between thumb and finger wearing nitrile gloves. 1.000 ml of 2 wt% polymer (grafted M280 or non-grafted M280) in water was evenly spread on the hair tress with a pipette and left for 1 h in air at room temperature. The hair tress was immersed for 20 s in 200 ml of 0.1 mol/l acetate buffer at pH 5 at room temperature and shaken gently to wash out excess polymer solution. The hair tress was rinsed thoroughly for 20 s with tap-water at 38°C. The hair tress was toweled dry with fresh paper and dried with a hair dryer without combing.

### Example 7

### Determination of effect of polymer deposition and durability of effect

Sample tresses, treated with the different polymers, were assessed for initial conditioning effect and conditioning effect after washing by hand by a panel of x trained researchers.

The washing routine for determination of durability of effect of grafted M280 vs non-grafted M280 was performed by thoroughly wetting the tress for 20 s with tap-water at 38°C, applying deep-cleansing shampoo (Goldwell, KERASILK Control, 0.5 ml) on the wet hair tress, and rubbing with nitrile-gloved fingers. The deep-cleansing shampoo foam was thoroughly rinsed off for 20 s with tap-water at 38°C. Wet combing and wet feeling were evaluated. Each hair tress was toweled dry with fresh paper and dried with a hair dryer without combing. Dry combing and dry feeling were evaluated.

| | Washing cycle | | | | | |
|---|---|---|---|---|---|---|
| | Initial | 1x | 2x | 3x | 4x | 5x |
| Merquat 280 | * | * | * | * | * | * |
| Merquat 280-DA | **** | **** | **** | **** | *** | ** |

| | | | | | | |
|---|---|---|---|---|---|---|
| * indicates degree of conditioning effect: **** = Excellent, *** = Very Good, ** = Good, * = Acceptable | | | | | | |

The results above show that, upon initial deposition, the DA grafted Merquat 280 polymer exhibited a significantly higher conditioning effect to the hair than the unmodified Merquat 280 polymer. Upon washing, it can be seen that the performance of the grafted polymer was judged to outperform the unmodified polymer, even after five washing cycles.

The following examples comprises the derivatized polymers in order to provide long lasting conditioning hair.

### Example 8

| | % by weight |
|---|---|
| Sodium lauryl ether sulphate | 8.0 |
| Coco glucoside | 3.0 |
| Cocamidopropyl betaine | 3.0 |
| Polymer of Example 1 | 1.0 |
| Citric acid/sodium hydroxide | q.s. to pH 5.5 |
| Preservative, fragrance | q.s |
| Water | to 100 |

The above shampoo composition showed good conditioning effect on hair and the conditioning effect lasted for 3 hair washes with a shampoo composition not comprising the polymer of Example 1.

In an another test, hair washed with the above shampoo composition 3 consecutive times and afterwards the hair conditioning effect lasted for hair washes with a shampoo composition not comprising the polymer of Example 1.

### Example 9

| | % by weight |
|---|---|
| Sodium lauryl ether sulphate | 8.0 |
| Coco glucoside | 3.0 |
| Cocoamphoacetate | 4.0 |
| Polyquaternium-7 | 0.8 |
| Panthenol | 0.5 |
| Polymerof Example 1 | 1.0 |
| Basic red 51 | 0.1 |
| Lactic acid/sodium hydroxide | q.s. to pH 5.5 |
| Preservative, fragrance | q.s |
| Water | to 100 |

Above composition gives hair a red shine, in addition to the excellent softness.

### Example 10

| | % by weight |
|---|---|
| Dicocoylethylhydroxyethylmonium methosulfate | 0.5 |
| Behenyl alcohol | 5 |
| Ceteareth-20 | 2 |
| Polyquaternium-10 | 1 |
| Dimethicone | 0.5 |
| Polymer of Example 1 | 0.5 |
| Lactic acid | q.s. pH 4.2 |
| Fragrance, preservative | q.s. |
| Water | to 100 |

### Example 11

| | % by weight |
|---|---|
| Cetearyl alcohol | 7 |
| Ceteareth-20 | 3 |
| Benzophenone-4 | 0.2 |
| Isopropyl myristate | 0.2 |
| Glycerin | 3 |
| Guar hydroxyproly trimonium chloride | 0.3 |
| Polymer of Example 1 | 0.8 |
| Basic red 51 | 0.1 |
| Citric acid | q.s. pH 4.2 |
| Fragrance, preservative | q.s. |
| Water | to 100 |

### Example 12 Aerosol spray

| **Bulk** | % by weight |
|---|---|
| VP/VA copolymer | 7.5 |
| Polymer of example 1 | 0.75 |
| Fragrance | 0.2 |
| Sodium hydroxide | q.s. to pH 7.9 |
| Alcohol | q.s. to 100 |

### Aerosol:

| | |
|---|---|
| Bulk of above | 50 % by weight |
| Dimethylether | 50 % by weight |

### Example 13 Aerosol foam

| **Bulk** | % by weight |
|---|---|
| Ethanol | 10.0 |
| PVP (Luviskol K 90) | 7.3 |
| Polymer of Example 1 | 1.0 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Aerosol:

| | |
|---|---|
| Bulk of above | 90 % by weight |
| Propane butane | 10 % by weight |

## Claims

1. A compound comprising a polymeric backbone comprising one or more of reactive hydroxyl, carboxyl and/or primary or secondary amine groups and a deposition enhancing group, according to the general structure 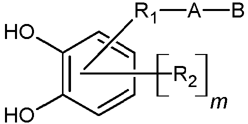 wherein
R₁ is C₁ to C₄ alkyl or C₂ to C₄ alkyl, which may be saturated or unsaturated and/or substituted with one or more groups excluding a primary, secondary or tertiary amine group, and a COOH group or C₃ to C₄ branched alkyl, which may be saturated or unsaturated and/or substituted with one or more groups excluding a primary, secondary or tertiary amine group, and a COOH group, A is a carboxyl or a primary amine group,
B is a polymeric backbone carrying one or more of hydroxyl, carboxyl and/or primary or secondary amine group, R₂ is OH, C₁ to C₄ alkyl, C₂ to C₄ alkyl, which may be saturated or unsaturated and/or substituted with one or more groups excluding a primary, secondary or tertiary amine group, and a COOH group and C₃ to C₄ branched alkyl, which may be saturated or unsaturated and/or substituted with one or more hydroxyl groups excluding a primary, secondary or tertiary amine group, and a COOH group and m is 0 to 3,
wherein 1 to 50% of the total reactive hydroxyl, carboxyl and/or primary or secondary amine groups on the polymeric backbone are occupied with deposition enhancing group,
with the condition that the A is a carboxyl group when the polymeric backbone B has primary and/or secondary amine groups as the reactive group or hydroxyl group and A is primary amine when the polymeric backbone B has carboxyl groups as the reactive group.

2. The compound according to claim 1 wherein R1 is C2 alkyl (ethyl) and m is 0.

3. The compound according to claims 1 and/or 2 wherein polymeric backbone is selected from the polymers which are freely soluble in a solvent preferably selected from dimethylformamide, dichlororomethane and water and their mixture.

4. The compound according to claims 1 to 3 wherein polymeric backbone is selected from the polymers which may be homo or copolymer and having one or more monomer units selected from acrylic acid, methacrylic acid, ethacrylic acid, methyl acrylate, ethyl acrylate, n-butyl acrylate, iso-butyl acrylate, t-butyl acrylate, 2-ethylhexyl acrylate, decyl acrylate, octyl acrylate, methyl methacrylate, ethyl methacrylate, n-butyl methacrylate, iso-butyl methacrylate, t-butyl methacrylate, 2-ethylhexyl methacrylate, decyl methacrylate, methyl ethacrylate, ethyl ethacrylate, n-butyl ethacrylate, iso-butyl ethacrylate, t-butyl ethacrylate, 2-ethylhexyl ethacrylate, decyl ethacrylate, 2,3-dihydroxypropyl acrylate, 2,3-dihydroxypropyl methacrylate, 2-hydroxyethyl acrylate, 2-hydroxypropyl acrylate, hydroxypropyl methacrylate, glyceryl monoacrylate, glyceryl monoethacrylate, glycidyl methacrylate, glycidyl acrylate, acrylamide, methacrylamide, ethacrylamide, N-methyl acrylamide, N,N-dimethyl acrylamide, N,N-dimethyl methacrylamide, N-ethyl acrylamide, N-isopropyl acrylamide, N-butyl acrylamide, N-t-butyl acrylamide, N,N-di-n-butyl acrylamide, N,N-diethylacrylamide, N-octyl acrylamide, N-octadecyl acrylamide, N,N-diethylacrylamide, N-phenyl acrylamide, N-methyl methacrylamide, N-ethyl methacrylamide, N-dodecyl methacrylamide, N,N-dimethylaminoethyl acrylamide, quaternized N,N-dimethylaminoethyl acrylamide, N,N-dimethylaminoethyl methacrylamide, quaternized N,N-dimethylaminoethyl methacrylamide, N,N-dimethylaminoethyl acrylate, N,N-dimethylaminoethyl methacrylate, quaternized N,N-dimethyl-aminoethyl acrylate, quaternized N,N-dimethylaminoethyl methacrylate, 2-hydroxyethyl acrylate, 2-hydroxyethyl methacrylate, 2-hydroxyethyl ethacrylate, glyceryl acrylate, 2-methoxyethyl acrylate, 2-methoxyethyl methacrylate, 2-methoxyethyl ethacrylate, 2-ethoxyethyl acrylate, 2-ethoxyethyl methacrylate, 2-ethoxyethyl ethacrylate, maleic acid, maleic anhydride and its half esters, fumaric acid, itaconic acid, itaconic anhydride and its half esters, crotonic acid, angelic acid, diallyldimethyl ammonium chloride, vinyl pyrrolidone, vinyl imidazole, methyl vinyl ether, methyl vinyl ketone, maleimide, vinyl pyridine, vinyl furan, styrene sulphonate, allyl alcohol, allyl citrate, allyl tartrate, vinyl acetate, vinyl alcohol, and mixtures thereof, silicones with isopropyl, propyl or butyl amino propyl amine groups which may be grafted with an oxazoline comprising groups, and heteropolysaccharide gum.

5. The compound according to claims 1 to 4 wherein the deposition enhancing group is selected from dihydroxyphenylethylamine, dihydroxyphenyl propionic acid, norepinephrine, hydroxytyrosol and caffeic acid, preferably it is dihydroxyphenylethylamine or dihydroxyphenyl propionic acid.

6. The compound according to any of the claims 1 to 5 is a hair-conditioning compound.

7. The compound according to claims 1 to 6 which is one of the compounds according to structures 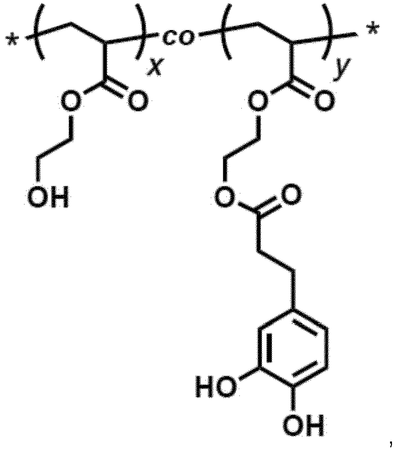 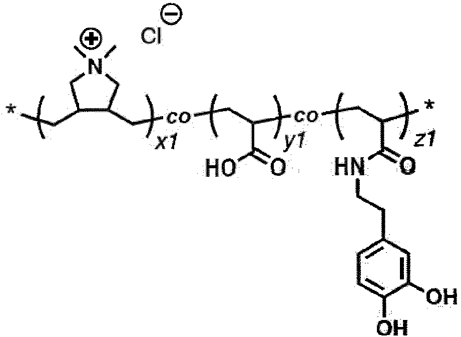 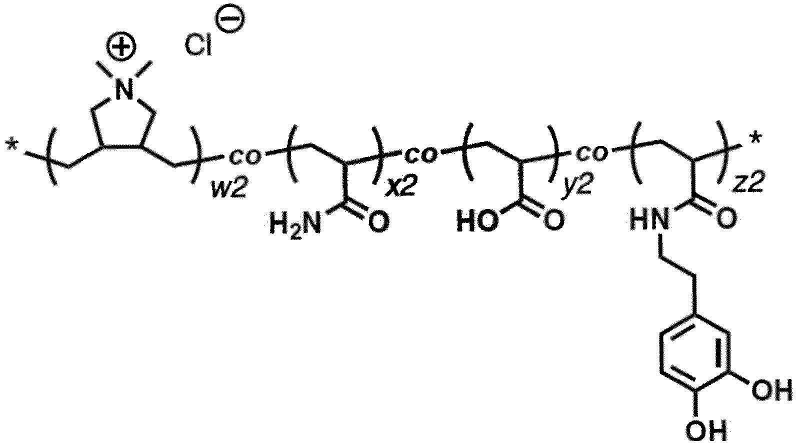 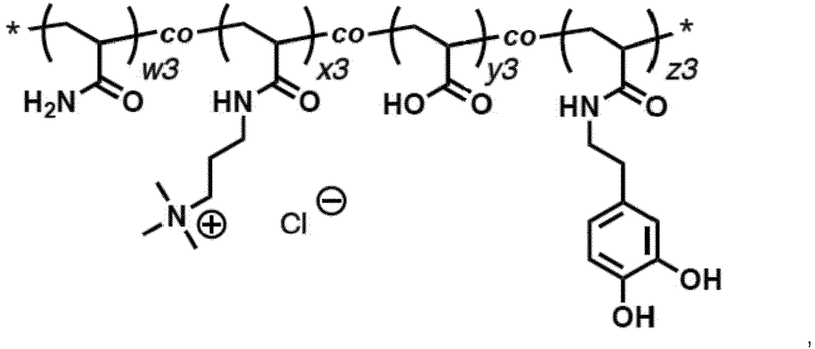 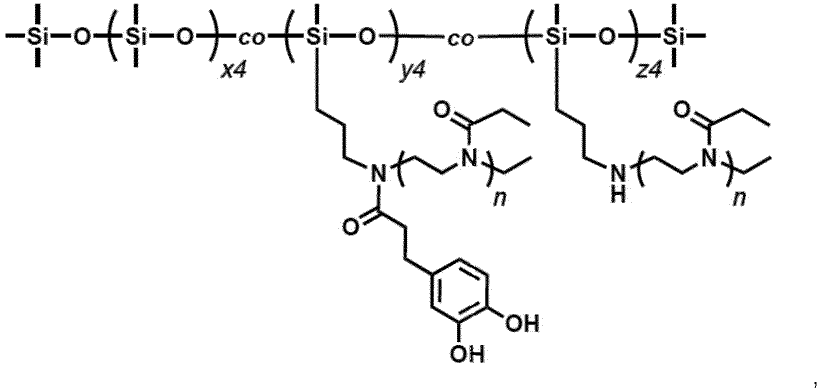 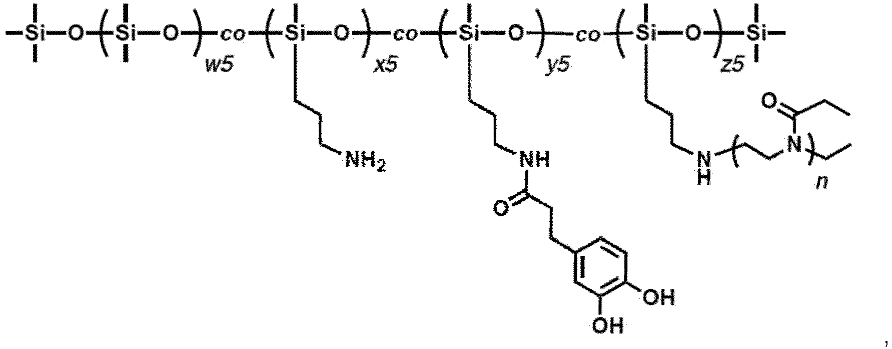 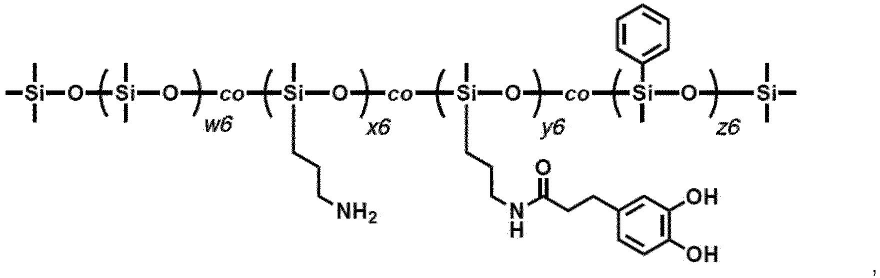 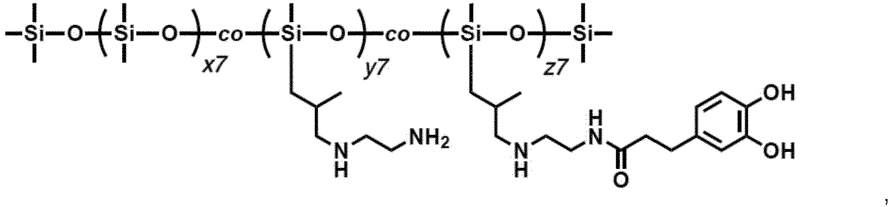 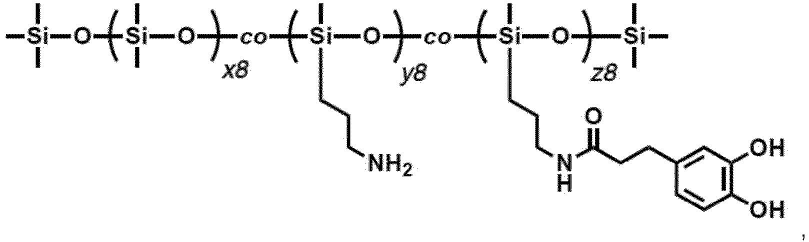 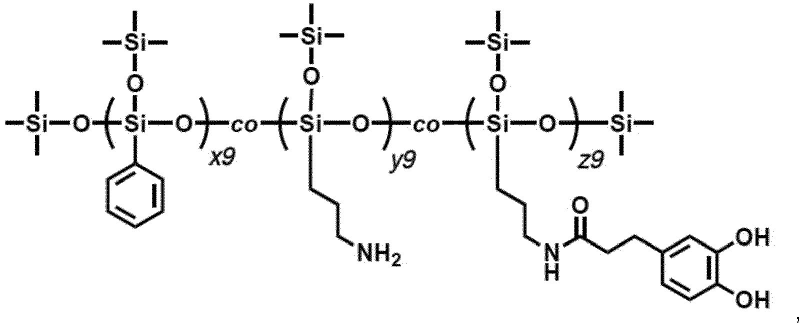 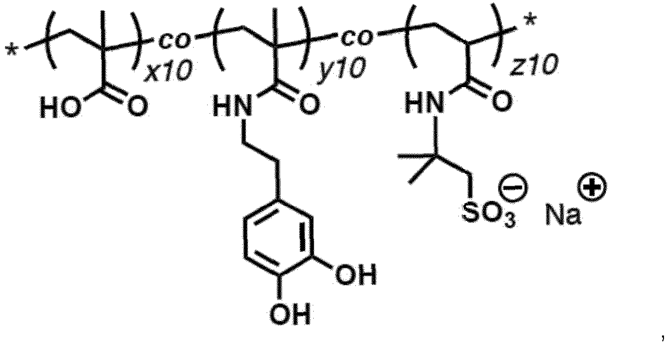 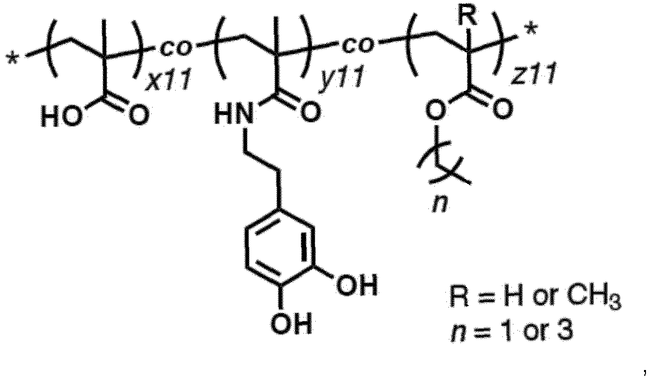 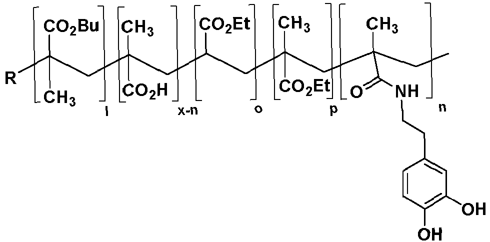 and

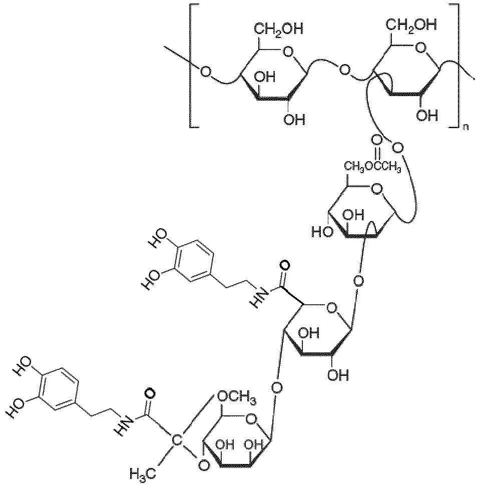

8. Method of synthesizing the compounds of according to any of the claims 1 to 7, comprising the steps of
a- the polymer and the deposition enhancing dihydroxy compound are dissolved in a solvent or mixture of solvents, preferably selected from dichloromethane, N,N-dimethyl formamide, water and their mixture, and
b- reacted for a period of 1 h to 7 days, preferably 6 hrs to 5 days at a temperature in the range of 20 to 60°C, in the presence of one or more coupling agents selected from EDC, NHS, PyBOP, HBTU, HOBt, DCC, DIC and their mixture, and optionally a catalyst selected from DIPEA and DMAP, and
c- optionally, the derivatized polymer is precipitated by changing the solvent to a solvent wherein the derivatized polymer is insoluble, preferably selected from Diethylether and/or acetone, and
d- the derivatized polymer is dissolved in a solvent or mixture of solvents preferably selected from dichloromethane, N,N-dimethyl formamide, water and their mixture and dialyzed against methanol or 1mM hydrochloric acid solution, and
e- the dry purified derivatized polymer is obtained by freeze drying,
with the conditions,
when to be derivatized polymer comprises carboxyl groups as the reactive group, the coupling agent is selected from EDC and/or NHS, and the reaction was carried out without a catalyst,
when to be derivatized polymer comprises primary or secondary amine groups as the reactive group, the coupling agent is selected from PyBOP, HBTU, HOBt, DCC, DIC, EDC and their mixture and the catalyst is DIPEA, and
when to be derivatized polymer comprises hydroxyl groups as the reactive group, the coupling agent is DCC, and the catalyst is DMAP.

9. The method according to claim 8 wherein the solvent is a mixture of dichloromethane, N,N-dimethyl formamide and water.

10. A cosmetic composition **characterized in that** it comprises one or more compounds of claims 1 to 7 or one or more compounds obtained by the method according to claims 8 to 9, preferably at a concentration in the range of 0.1 to 5%, further preferably 0.1 to 4%, more preferably 0.15 to 3% and most preferably 0.2 to 2.5% by weight, calculated to the total of the composition

11. The composition according to claim 10 **characterized in that** it comprises one or more of surfactants selected from anionic, cationic, nonionic and amphoteric ones.

12. The composition according to claims 10 or 11 wherein it is an aqueous composition.

13. The composition according to claims 10 to 12 comprising the polymers with the deposition-enhancing group are in the form of shampoo, conditioner, hair spray, gel, colouring composition, permanent and/or semipermanent shaping composition, styling composition, aerosol, and lotion.

14. A cosmetic treatment method wherein the composition according to any of the claims 10 to 13 is applied onto hair and left on the hair for a period and optionally rinsed off from hair.

15. The method according to claim 14 **characterized in that** the hair is heated up to a temperature in the range of 40 to 230°C in the whole or a portion of the period wherein the composition is left on the hair.

16. Kit for hair **characterized in that** it comprises one or more cosmetic compositions according to claims 10 to 13.

## Patentansprüche

1. Verbindung, umfassend ein polymeres Gerüst, umfassend eine oder mehrere von reaktiven Hydroxyl-, Carboxyl- und/oder primären oder sekundären Aminogruppen sowie eine abscheidungsfördernde Gruppe gemäß der allgemeinen Struktur 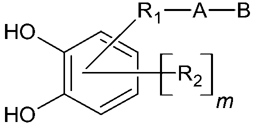 worin
R₁ C₁- bis C₄-Alkyl oder C₂- bis C₄-Alkyl, das gesättigt oder ungesättigt sein kann und/oder mit einer oder mehreren Gruppen, ausgenommen einer primären, sekundären oder tertiären Aminogruppe und einer COOH-Gruppe, substituiert sein kann, oder verzweigtes C₃- bis C₄-Alkyl, das gesättigt oder ungesättigt sein kann und/oder mit einer oder mehreren Gruppen, ausgenommen einer primären, sekundären oder tertiären Aminogruppe und einer COOH-Gruppe, substituiert sein kann, ist,
A eine Carboxyl- oder eine primäre Aminogruppe ist,
B ein polymeres Gerüst ist, das eine oder mehrere von einer Hydroxyl-, Carboxyl- und/oder primären oder sekundären Aminogruppe trägt, R₂ OH, C₁- bis C₄-Alkyl, C₂- bis C₄-Alkyl, das gesättigt oder ungesättigt sein kann und/oder mit einer oder mehreren Gruppen, ausgenommen einer primären, sekundären oder tertiären Aminogruppe und einer COOH-Gruppe, substituiert sein kann, und verzweigtes C₃- bis C₄-Alkyl, das gesättigt oder ungesättigt sein kann und/oder mit einer oder mehreren Hydroxylgruppen, ausgenommen einer primären, sekundären oder tertiären Aminogruppe und einer COOH-Gruppe, substituiert sein kann, und m 0 bis 3 ist,
wobei 1 bis 50% der gesamten reaktiven Hydroxyl-, Carboxyl- und/oder primären oder sekundären Aminogruppen an dem polymeren Gerüst mit der abscheidungsfördernden Gruppe besetzt sind,
mit der Maßgabe, dass A eine Carboxylgruppe ist, wenn das polymere Gerüst B primäre und/oder sekundäre Aminogruppen als reaktive Gruppe oder Hydroxylgruppe aufweist, und A ein primäres Amin ist, wenn das polymere Gerüst B Carboxylgruppen als reaktive Gruppe aufweist.

2. Verbindung gemäß Anspruch 1, wobei R1 C2-Alkyl (Ethyl) ist und m 0 ist.

3. Verbindung gemäß den Ansprüchen 1 und/oder 2, wobei das polymere Gerüst aus Polymeren ausgewählt ist, die in einem Lösungsmittel, bevorzugt ausgewählt aus Dimethylformamid, Dichlormethan und Wasser sowie Mischungen davon, frei löslich sind.

4. Verbindung gemäß den Ansprüchen 1 bis 3, wobei das polymere Gerüst aus Polymeren ausgewählt ist, die Homo- oder Copolymere und mit einer oder mehreren Monomereinheiten, ausgewählt aus Acrylsäure, Methacrylsäure, Ethacrylsäure, Methylacrylat, Ethylacrylat, n-Butylacrylat, Isobutylacrylat, t-Butylacrylat, 2-Ethylhexylacrylat, Decylacrylat, Octylacrylat, Methylmethacrylat, Ethylmethacrylat, n-Butylmethacrylat, Isobutylmethacrylat, t-Butylmethacrylat, 2-Ethylhexylmethacrylat, Decylmethacrylat, Methylethacrylat, Ethylethacrylat, n-Butylethacrylat, Isobutylethacrylat, t-Butylethacrylat, 2-Ethylhexylethacrylat, Decylethacrylat, 2,3-Dihydroxypropylacrylat, 2,3-Dihydroxypropylmethacrylat, 2-Hydroxyethylacrylat, 2-Hydroxypropylacrylat, Hydroxypropylmethacrylat, Glycerylmonoacrylat, Glycerylmonoethacrylat, Glycidylmethacrylat, Glycidylacrylat, Acrylamid, Methacrylamid, Ethacrylamid, N-Methylacrylamid, N,N-Dimethylacrylamid, N,N-Dimethylmethacrylamid, N-Ethylacrylamid, N-Isopropylacrylamid, N-Butylacrylamid, N-t-Butylacrylamid, N,N-Di-n-butylacrylamid, N,N-Diethylacrylamid, N-Octylacrylamid, N-Octadecylacrylamid, N,N-Diethylacrylamid, N-Phenylacrylamid, N-Methylmethacrylamid, N-Ethylmethacrylamid, N-Dodecylmethacrylamid, N,N-Dimethylaminoethylacrylamid, quaternisiertem N,N-Dimethylaminoethylacrylamid, N,N-Dimethylaminoethylmethacrylamid, quaternisiertem N,N-Dimethylaminoethylmethacrylamid, N,N-Dimethylaminoethylacrylat, N,N-Dimethylaminoethylmethacrylat, quaternisiertem N,N-Dimethylaminoethylacrylat, quaternisiertem N,N-Dimethylaminoethylmethacrylat, 2-Hydroxyethylacrylat, 2-Hydroxyethylmethacrylat, 2-Hydroxyethylethacrylat, Glycerylacrylat, 2-Methoxyethylacrylat, 2-Methoxyethylmethacrylat, 2-Methoxyethylethacrylat, 2-Ethoxyethylacrylat, 2-Ethoxyethylmethacrylat, 2-Ethoxyethylethacrylat, Maleinsäure, Maleinsäureanhydrid und Halbester davon, Fumarsäure, Itaconsäure, Itaconsäureanhydrid und Halbester davon, Crotonsäure, Angelinsäure, Diallyldimethylammoniumchlorid, Vinylpyrrolidon, Vinylimidazol, Methylvinylether, Methylvinylketon, Maleimid, Vinylpyridin, Vinylfuran, Styrolsulfonat, Allylalkohol, Allylcitrat, Allyltartrat, Vinylacetat, Vinylalkohol und Mischungen davon, Silikone mit Isopropyl-, Propyl- oder Butylaminopropylaminogruppen, die mit Oxazolin umfassenden Gruppen gepfropft sein können, und Heteropolysaccharidgummi sein können.

5. Verbindung gemäß den Ansprüchen 1 bis 4, wobei die abscheidungsfördernde Gruppe aus Dihydroxyphenylethylamin, Dihydroxyphenylpropionsäure, Noradrenalin, Hydroxytyrosol und Coffeinsäure ausgewählt ist, es bevorzugt Dihydroxyphenylethylamin oder Dihydroxyphenylpropionsäure ist.

6. Verbindung gemäß einem der Ansprüche 1 bis 5, die eine Haarpflegeverbindung ist.

7. Verbindung gemäß den Ansprüchen 1 bis 6, die eine der Verbindungen gemäß den Strukturen 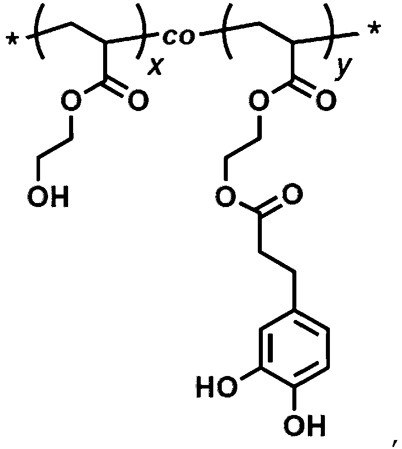 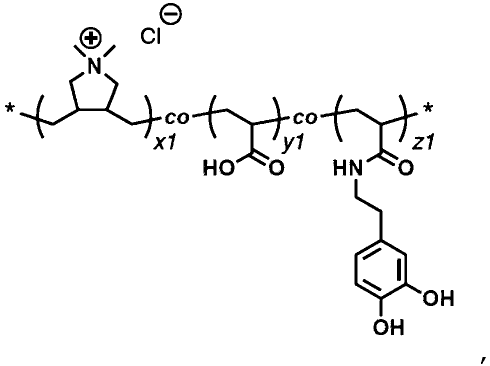 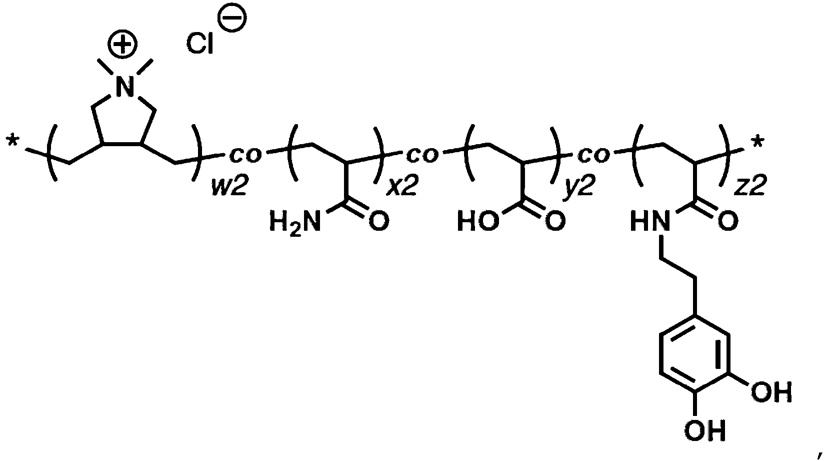 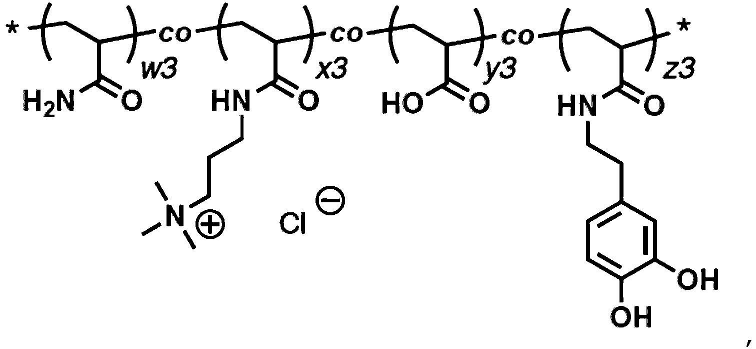 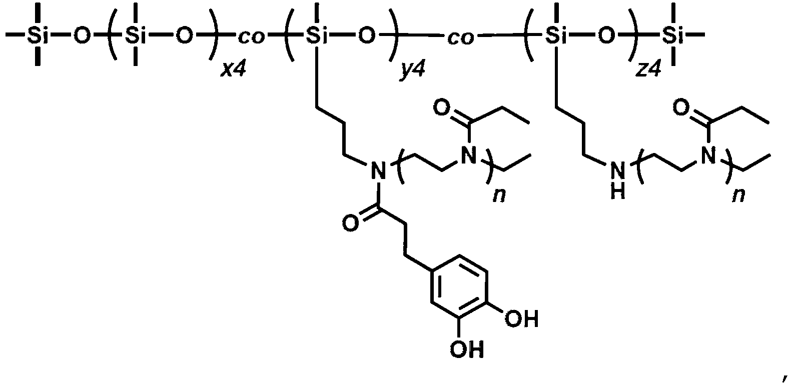 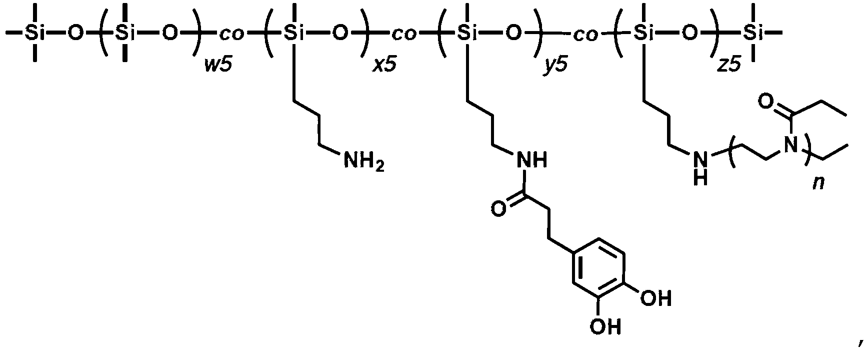 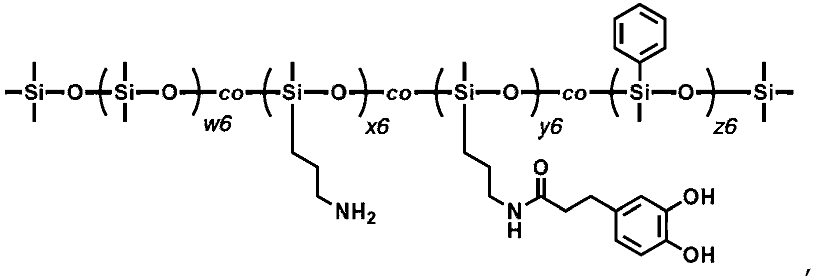 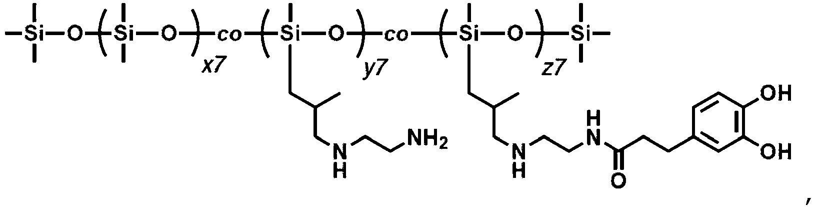 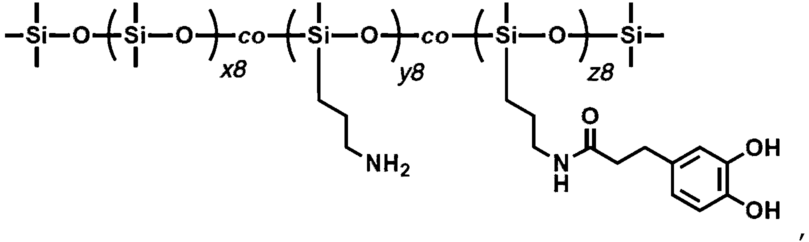 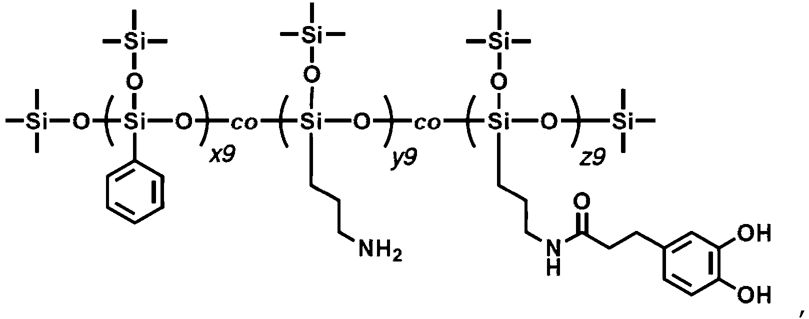 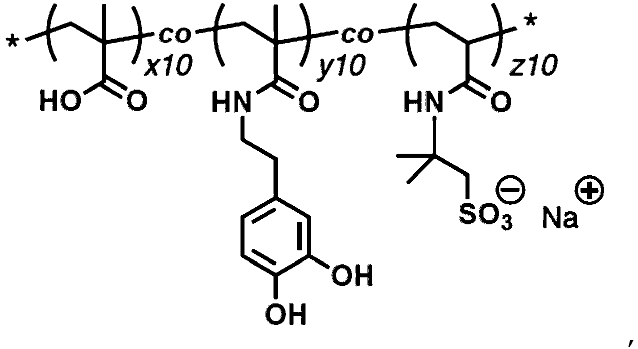 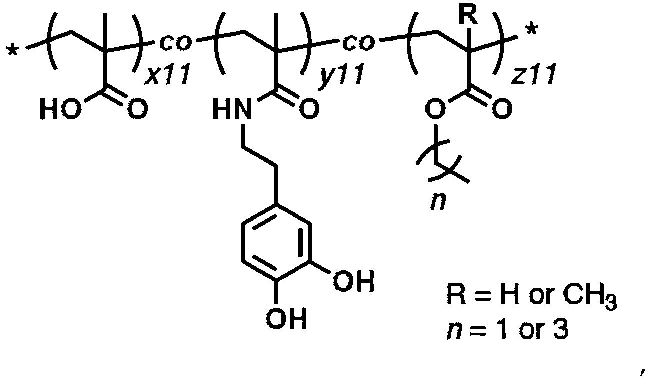 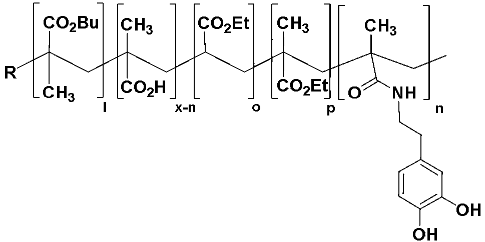 und 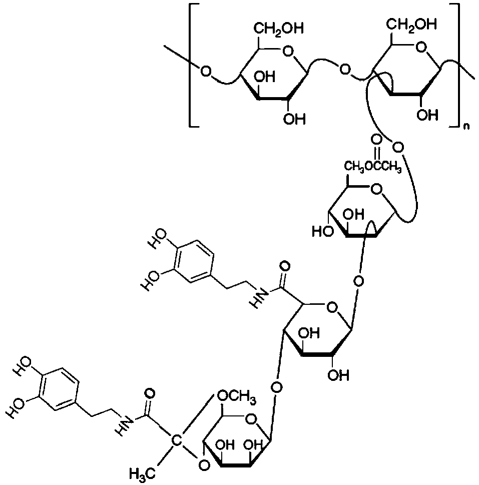 ist.

8. Verfahren zur Synthese der Verbindungen gemäß einem der Ansprüche 1 bis 7, umfassend die Schritte:
a- das Polymer und die abscheidungsfördernde Dihydroxyverbindung werden in einem Lösungsmittel oder einem Lösungsmittelgemisch, bevorzugt ausgewählt aus Dichlormethan, N,N-Dimethylformamid, Wasser und Mischungen davon, gelöst, und
b- über einen Zeitraum von 1 Stunde bis 7 Tagen, bevorzugt 6 Stunden bis 5 Tagen, bei einer Temperatur im Bereich von 20 bis 60°C in Gegenwart eines oder mehrerer Kupplungsmittel, ausgewählt aus EDC, NHS, PyBOP, HBTU, HOBt, DCC, DIC und Mischungen davon, sowie optional eines Katalysators, ausgewählt aus DIPEA und DMAP, zur Reaktion gebracht, und
c- optional wird das derivatisierte Polymer durch Wechsel des Lösungsmittels zu einem Lösungsmittel, in dem das derivatisierte Polymer unlöslich ist, bevorzugt ausgewählt aus Diethylether und/oder Aceton, gefällt, und
d- das derivatisierte Polymer wird in einem Lösungsmittel oder einem Lösungsmittelgemisch, bevorzugt ausgewählt aus Dichlormethan, N,N-Dimethylformamid, Wasser und Mischungen davon, gelöst und gegen Methanol oder eine 1 mM Salzsäurelösung dialysiert, und
e- das trockene, gereinigte derivatisierte Polymer wird durch Gefriertrocknung gewonnen,
mit den Bedingungen, dass
wenn das zu derivatisierende Polymer Carboxylgruppen als reaktive Gruppe umfasst, das Kupplungsmittel aus EDC und/oder NHS ausgewählt wird, und die Reaktion ohne Katalysator durchgeführt wurde,
wenn das zu derivatisierende Polymer primäre oder sekundäre Aminogruppen als reaktive Gruppe umfasst, das Kupplungsmittel aus PyBOP, HBTU, HOBt, DCC, DIC, EDC und Mischungen davon ausgewählt ist und der Katalysator DIPEA ist, und
wenn das zu derivatisierende Polymer Hydroxylgruppen als reaktive Gruppe umfasst, das Kupplungsmittel DCC ist und der Katalysator DMAP ist.

9. Verfahren gemäß Anspruch 8, wobei das Lösungsmittel ein Gemisch aus Dichlormethan, N,N-Dimethylformamid und Wasser ist.

10. Kosmetische Zusammensetzung, **dadurch gekennzeichnet, dass** sie eine oder mehrere Verbindungen gemäß den Ansprüchen 1 bis 7 oder eine oder mehrere Verbindungen, erhalten durch das Verfahren gemäß den Ansprüchen 8 bis 9, bevorzugt in einer Konzentration im Bereich von 0,1 bis 5 Gew.-%, weiter bevorzugt 0,1 bis 4 Gew.-%, mehr bevorzugt 0,15 bis 3 Gew.-% und am meisten bevorzugt 0,2 bis 2,5 Gew.-%, berechnet auf die Gesamtheit der Zusammensetzung, umfasst.

11. Zusammensetzung gemäß Anspruch 10, **dadurch gekennzeichnet, dass** sie ein oder mehrere Tenside umfasst, die aus anionischen, kationischen, nichtionischen und amphoteren Tensiden ausgewählt sind.

12. Zusammensetzung gemäß Anspruch 10 oder 11, wobei sie eine wässrige Zusammensetzung ist.

13. Zusammensetzung gemäß den Ansprüchen 10 bis 12, umfassend die Polymere mit der abscheidungsfördernden Gruppe, die in Form von Shampoo, Conditioner, Haarspray, Gel, Färbezusammensetzung, permanenter und/oder semipermanenter Formungszusammensetzung, Stylingzusammensetzung, Aerosol und Lotion sind.

14. Kosmetisches Behandlungsverfahren, wobei die Zusammensetzung gemäß einem der Ansprüche 10 bis 13 auf das Haar aufgetragen und für eine Zeit auf dem Haar belassen und optional vom Haar abgespült wird.

15. Verfahren gemäß Anspruch 14, **dadurch gekennzeichnet, dass** das Haar während der gesamten oder eines Teils der Zeit, in der die Zusammensetzung auf dem Haar belassen wird, auf eine Temperatur im Bereich von 40 bis 230°C erwärmt wird.

16. Kit für Haar, **dadurch gekennzeichnet, dass** es eine oder mehrere kosmetische Zusammensetzungen gemäß den Ansprüchen 10 bis 13 umfasst.

## Revendications

1. Composé comprenant une chaîne principale polymère comprenant un ou plusieurs groupes parmi des groupes hydroxyle, carboxyle et/ou amine primaire ou secondaire réactifs et un groupe améliorant le dépôt, selon la structure générale 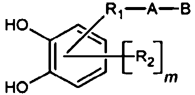 dans lequel
R₁ est un groupe alkyle en C₁ à C₄ ou un groupe alkyle en C₂ à C₄, qui peut être saturé ou insaturé et/ou substitué par un ou plusieurs groupes à l'exclusion d'un groupe amine primaire, secondaire ou tertiaire, et un groupe COOH ou un groupe alkyle ramifié en C₃ à C₄, qui peut être saturé ou insaturé et/ou substitué par un ou plusieurs groupes à l'exclusion d'un groupe amine primaire, secondaire ou tertiaire, et un groupe COOH,
A est un groupe carboxyle ou un groupe amine primaire,
B est une chaîne principale polymère portant un ou plusieurs groupes parmi des groupes hydroxyle, carboxyle et/ou
amine primaire ou secondaire, R₂ est OH, un groupe alkyle en C₁ à C₄, un groupe alkyle en C₂ à C₄, qui peut être saturé ou insaturé et/ou substitué par un ou plusieurs groupes à l'exclusion d'un groupe amine primaire, secondaire ou tertiaire, et un groupe COOH et un groupe alkyle ramifié en C₃ à C₄, qui peut être saturé ou insaturé et/ou substitué par un ou plusieurs groupes hydroxyle à l'exclusion d'un groupe amine primaire, secondaire ou tertiaire, et un groupe COOH et m est 0 à 3,
dans lequel 1 à 50 % du total des groupes hydroxyle, carboxyle et/ou amine primaire ou secondaire réactifs sur la chaîne principale polymère sont occupés par un groupe améliorant le dépôt,
à la condition que A soit un groupe carboxyle lorsque la chaîne principale polymère B présente des groupes amine primaire et/ou secondaire comme groupe réactif ou groupe hydroxyle et qu'A soit un amine primaire lorsque la chaîne principale polymère B présente des groupes carboxyles comme groupe réactif.

2. Composé selon la revendication 1 dans lequel R1 est un groupe alkyle en C2 (éthyl) et m est 0.

3. Composé selon les revendications 1 et/ou 2 dans lequel la chaîne principale polymère est sélectionnée parmi les polymères qui sont librement solubles dans un solvant préférablement sélectionné parmi le diméthylformamide, le dichlorométhane et l'eau et leur mélange.

4. Composé selon les revendications 1 à 3 dans lequel la chaîne principale polymère est sélectionnée parmi les polymères qui peuvent être des homopolymères ou des copolymères et présentant un ou plusieurs motifs monomères sélectionnés parmi l'acide acrylique, l'acide méthacrylique, l'acide éthacrylique, l'acrylate de méthyle, l'acrylate d'éthyle, l'acrylate de n-butyle, l'acrylate d'iso-butyle, l'acrylate de t-butyle, l'acrylate de 2-éthylhexyle, l'acrylate de décyle, l'acrylate d'octyle, le méthacrylate de méthyle, le méthacrylate d'éthyle, le méthacrylate de n-butyle, le méthacrylate d'iso-butyle, le méthacrylate de t-butyle, le méthacrylate de 2-éthylhexyle, le méthacrylate de décyle, l'éthacrylate de méthyle, l'éthacrylate d'éthyle, l'éthacrylate de n-butyle, l'éthacrylate d'iso-butyle, l'éthacrylate de t-butyle, l'éthacrylate de 2-éthylhexyle, l'éthacrylate de décyle, l'acrylate de 2,3-dihydroxypropyle, le méthacrylate de 2,3-dihydroxypropyle, l'acrylate de 2-hydroxyéthyle, l'acrylate de 2-hydroxypropyle, le méthacrylate d'hydroxypropyle, le monoacrylate de glycéryle, le monoéthacrylate de glycéryle, le méthacrylate de glycidyle, l'acrylate de glycidyle, l'acrylamide, le méthacrylamide, l'éthacrylamide, l'acrylamide de N-méthyle, l'acrylamide de N,N-diméthyle, le méthacrylamide de N,N-diméthyle, l'acrylamide de N-éthyle, l'acrylamide de N-isopropyle, l'acrylamide de N-butyle, l'acrylamide de N-t-butyle, l'acrylamide de N,N-di-n-butyle, le N,N-diéthylacrylamide, l'acrylamide de N-octyle, l'acrylamide de N-octadécyle, le N,N-diéthylacrylamide, l'acrylamide de N-phényle, le méthacrylamide de N-méthyle, le méthacrylamide de N-éthyle, le méthacrylamide de N-dodécyle, l'acrylamide de N,N-diméthylaminoéthyle, l'acrylamide de N,N-diméthylaminoéthyle quaternisé, le méthacrylamide de N,N-diméthylaminoéthyle, le méthacrylamide de N,N-diméthylaminoéthyle quaternisé, l'acrylate de N,N-diméthylaminoéthyle, le méthacrylate de N,N-diméthylaminoéthyle, l'acrylate de N,N-diméthyl-aminoéthyle quaternisé, le méthacrylate de N,N-diméthylaminoéthyle quaternisé, l'acrylate de 2-hydroxyéthyle, le méthacrylate de 2-hydroxyéthyle, l'éthacrylate de 2-hydroxyéthyle, l'acrylate de glycéryle, l'acrylate de 2-méthoxyéthyle, le méthacrylate de 2-méthoxyéthyle, l'éthacrylate de 2-méthoxyéthyle, l'acrylate de 2-éthoxyéthyle, le méthacrylate de 2-éthoxyéthyle, l'éthacrylate de 2-éthoxyéthyle, l'acide maléique, l'anhydride maléique et ses demi-esters, l'acide fumarique, l'acide itaconique, l'anhydride itaconique et ses demi-esters, l'acide crotonique, l'acide angélique, le chlorure de diallyldiméthylammonium, la vinylpyrrolidone, le vinylimidazole, l'éther méthylvinylique, la cétone méthylvinylique, le maléimide, la vinylpyridine, le vinylfurane, le sulfonate de styrène, l'alcool allylique, le citrate d'allyle, le tartrate d'allyle, l'acétate de vinyle, l'alcool vinylique, et des mélanges de ceux-ci, des silicones présentant des groupes amine aminopropyle isopropyle, propyle ou butyle qui peuvent être greffés avec des groupes comprenant une oxazoline, et de la gomme d'hétéropolysaccharide.

5. Composé selon les revendications 1 à 4 dans lequel le groupe améliorant le dépôt est sélectionné parmi la dihydroxyphényléthylamine, l'acide dihydroxyphénylpropanoïque, la noradrénaline, l'hydroxytyrosol et l'acide caféique, de préférence il est la dihydroxyphényléthylamine ou l'acide dihydroxyphénylpropionique.

6. Composé selon l'une quelconque des revendications 1 à 5, qui est un composé d'après-shampooing.

7. Composé selon les revendications 1 à 6 qui est l'un des composés selon les structures. 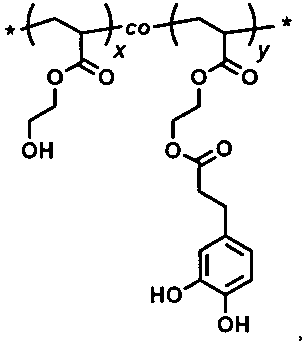 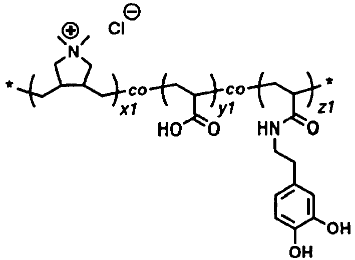 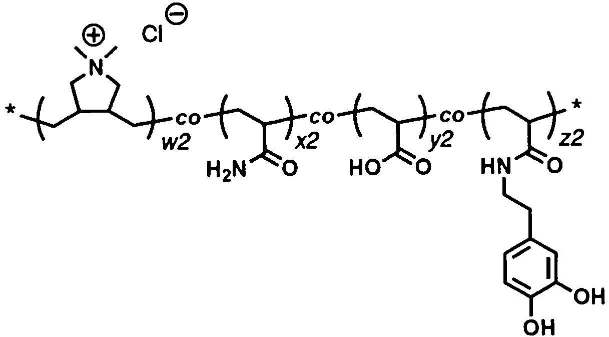 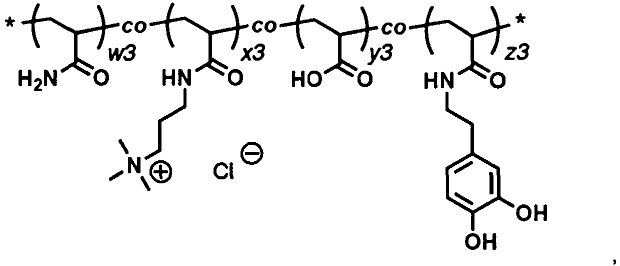 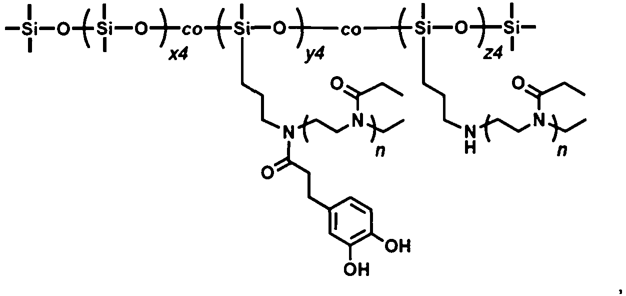 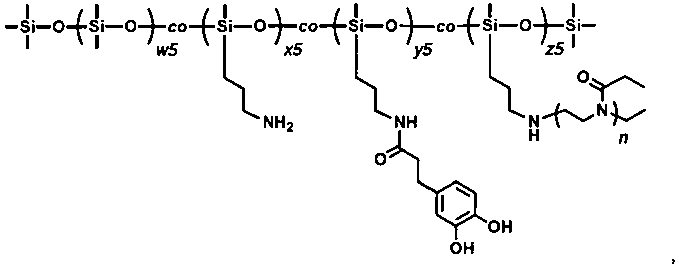 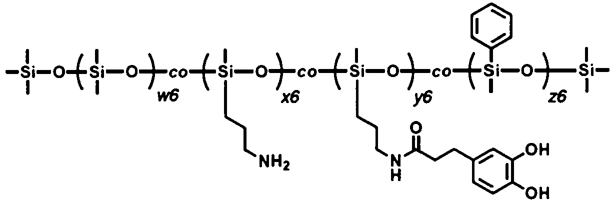 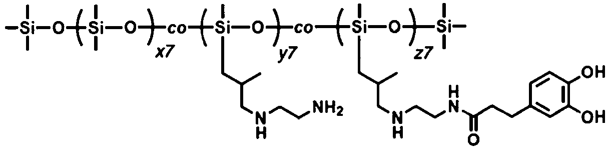 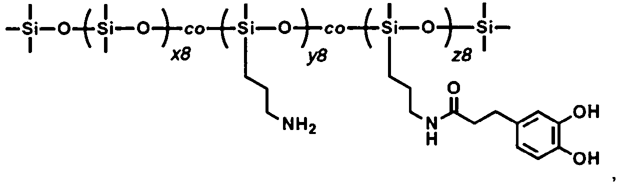 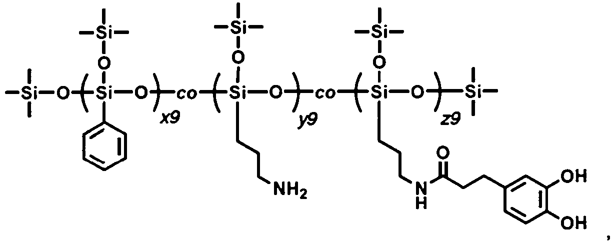 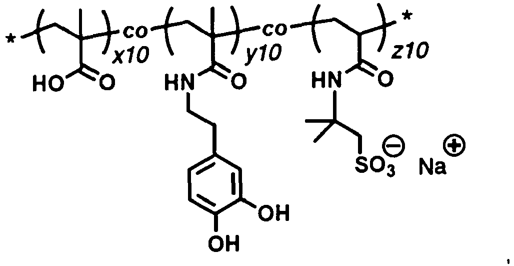 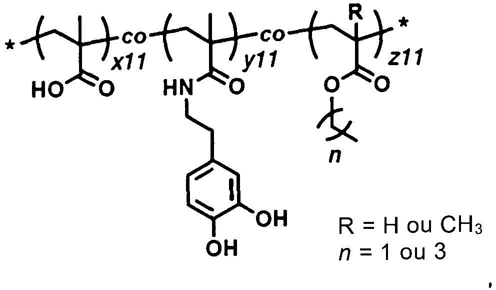 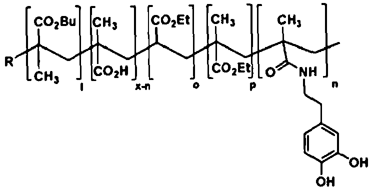 et

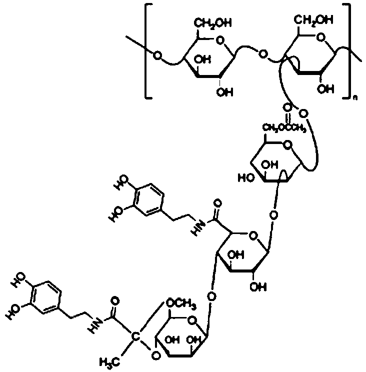

8. Procédé de synthèse des composés selon l'une quelconque des revendications 1 à 7, comprenant les étapes consistant en
a- le polymère et le composé dihydroxy améliorant le dépôt sont dissous dans un solvant ou un mélange de solvants, préférablement sélectionné parmi le dichlorométhane, le N,N-diméthylformamide, l'eau et leur mélange, et
b- une mise en réaction pendant une période de 1 h à 7 jours, préférablement 6 h à 5 jours à une température dans la plage de 20 à 60 °C, en présence d'un ou plusieurs agents de couplage sélectionnés parmi EDC, NHS, PyBOP, HBTU, HOBt, DCC, DIC et leur mélange, et, facultativement, un catalyseur sélectionné parmi la DIPEA et la DMAP, et
c- facultativement, le polymère dérivatisé est précipité en changeant le solvant en un solvant dans lequel le polymère dérivatisé est insoluble, préférablement sélectionné parmi l'éther diéthylique et/ou l'acétone, et
d- le polymère dérivatisé est dissous dans un solvant ou un mélange de solvants préférablement sélectionné parmi le dichlorométhane, le N,N-diméthylformamide, l'eau et leur mélange et dialysé contre du méthanol ou une solution d'acide chlorhydrique 1 mM, et
e- le polymère dérivatisé sec et purifié est obtenu par lyophilisation,
les conditions suivantes étant remplies,
lorsque le polymère à dérivatiser comprend des groupes carboxyles comme groupe réactif, l'agent de couplage est sélectionné parmi EDC et/ou NHS, et la réaction a été mise en œuvre sans catalyseur,
lorsque le polymère à dérivatiser comprend des groupes amine primaire ou secondaire comme groupe réactif, l'agent de couplage est sélectionné parmi PyBOP, HBTU, HOBt, DCC, DIC, EDC et leur mélange et le catalyseur est la DIPEA, et
lorsque le polymère à dérivatiser comprend des groupes hydroxyle comme groupe réactif, l'agent de couplage est le DCC, et le catalyseur est la DMAP.

9. Procédé selon la revendication 8 dans lequel le solvant est un mélange de dichlorométhane, de N,N-diméthylformamide et d'eau.

10. Composition cosmétique **caractérisée en ce qu'**elle comprend un ou plusieurs composés selon les revendications 1 à 7 ou un ou plusieurs composés obtenus par le procédé selon les revendications 8 à 9, de préférence à une concentration dans la plage de 0,1 à 5 %, de façon encore préférée de 0,1 à 4 %, plus préférentiellement de 0,15 à 3 % et le plus préférentiellement de 0,2 à 2,5 % en poids, calculée par rapport au total de la composition.

11. Composition selon la revendication 10 **caractérisée en ce qu'**elle comprend un ou plusieurs tensioactifs sélectionnés parmi des tensioactifs anioniques, cationiques, non ioniques et amphotères.

12. Composition selon les revendications 10 ou 11 dans laquelle elle est une composition aqueuse.

13. Composition selon les revendications 10 à 12 comprenant les polymères avec le groupe améliorant le dépôt sont sous la forme de shampooing, d'après-shampooing, de laque pour cheveux, de gel capillaire, de composition de coloration, de composition de mise en forme permanente des cheveux et/ou composition de mise en forme semi-permanente des cheveux, de composition de coiffage, d'aérosol, et de lotion.

14. Procédé de traitement cosmétique dans lequel la composition selon l'une quelconque des revendications 10 à 13 est appliquée sur les cheveux et laissée sur les cheveux pendant une période et facultativement rincée des cheveux.

15. Procédé selon la revendication 14 **caractérisé en ce que** les cheveux sont chauffés jusqu'à une température dans la plage de 40 à 230 °C pendant toute ou une partie de la période dans laquelle la composition est laissée sur les cheveux.

16. Kit pour les cheveux **caractérisé en ce qu'**il comprend une ou plusieurs compositions cosmétiques selon les revendications 10 à 13.
